# EUROPEAN PATENT APPLICATION

(11) **EP 3 981 410 A1**
(43) Date of publication of application: **13.04.2022**
(21) Application number: 20306165.0
(22) Date of filing: 07.10.2020
(51) Int. Cl.: A61K 31/5575, A61K 31/197, A61K 31/222, A61K 31/4402, A61K 31/4439, A61P 31/14, A61K 31/4706

(54) **PROSTAGLANDIN RECEPTOR AGONISTS FOR USE IN THE TREATMENT OF A CORONAVIRUS INFECTION SUCH AS COVID-19**

(71) Applicant: Medetia, 75015 Paris (FR)
(72) Inventor: ANNEREAU, Jean-Philippe, 92160 Antony (FR); BRISENO-ROA, Luis, 75013 Paris (FR)
(74) Representative: Icosa

(57) **Abstract**

The present invention relates to prostaglandin receptor agonists, such as EP2 and/or EP4 agonists, preferably EP2 agonists, for treating a disease caused by a coronavirus infection, such as COVID-19, in a subject in need thereof, especially in a subject at risk to develop a severe form and/or a complication of said disease.

## Description

### FIELD OF INVENTION

The present invention relates to the treatment of a disease caused by a coronavirus infection, such as COVID-19. Especially, the present invention relates to prostaglandin receptor agonists for use in treating a disease caused by a coronavirus infection, such as COVID-19, in a subject in need thereof, especially in a subject at risk to develop a severe form and/or a complication of said disease. Preferably the prostaglandin receptor agonists increase cAMP levels, such as for example EP2 and/or EP4 agonists. The invention also relates to prostaglandin receptor agonists for use to induce an interferon-like response, by inducing one or more of genes related to interferon signaling pathway.

### BACKGROUND OF INVENTION

Coronaviruses (CoVs) are ribonucleic acid (RNA) viruses of the *Coronaviridae* family. Coronaviruses infect mammals and birds and cause a wide range of respiratory, gastrointestinal, neurologic, and systemic diseases. In most cases, human coronaviruses cause only mild respiratory infections, such as the common cold. However, in recent years, two highly pathogenic coronaviruses causing severe respiratory diseases emerged from animal reservoirs: severe acute respiratory syndrome coronavirus (SARS-CoV-1) first identified in 2003 and Middle East respiratory syndrome coronavirus (MERS-CoV) first identified in 2012.

In December 2019, the Wuhan Municipal Health Committee, China, identified a new infectious respiratory disease of unknown cause. Coronavirus RNA was quickly identified in some of the patients and in January 2020, a full genomic sequence of the newly identified human coronavirus SARS-CoV-2 (previously known as 2019-nCoV) was released by Shanghai Public Health Clinical Center & School of Public Health, Fudan University, Shanghai, China. The genomic sequence of SARS-COV-2 has 82% nucleotide identity with the genomic sequence of human SARS-CoV-1. Moreover, as previously shown for SARS-CoV-1, SARS-CoV-2 appears to utilize ACE2 (angiotensin converting enzyme 2) as receptor for viral cell entry (Hou et al., Cell, 2020, 182, 429-446).

In infected subjects exhibiting symptoms, the disease caused by SARS-COV-2 is now termed "coronavirus disease 2019" (COVID-19). COVID-19 is a respiratory illness with a broad clinical spectrum. The majority of affected subjects experience mild or moderate symptoms. COVID-19 generally presents first with symptoms including headache, muscle pain, fatigue, fever and respiratory symptoms (such as a dry cough, shortness of breath, and/or chest tightness). Other reported symptoms include a loss of smell (anosmia) and/or taste (ageusia). Some subjects develop a severe form of COVID-19 that may lead to pneumonitis and acute respiratory failure. Complications of COVID-19 also include thrombotic complications, pulmonary embolism, cardiovascular failure, renal failure, liver failure and secondary infections.

It is estimated that up to 25% of people around the world have at least one underlying condition that put them at increased risk of severe COVID-19 if they are infected and that about 5% of the global population are likely to require hospitalization if infected. The disease severity is tightly associated with age and the presence of comorbidities. Especially, mortality rates are above 15% for the elderly (+80y).

Besides, it was evidenced that when subjects suffering from COVID-19 have a low IFN-g response during the early stage response to COVID-19, it is associated to severe evolution of the disease and to complications, due to a massive cytokine storm counter reaction (Hadjadj et al., Science, 2020, 369, 718-724; Blanco-Melo et al., Cell, 2020, 181, 1036-1045).

Global efforts to identify an effective treatment for COVID-19 are ongoing. A number of clinical trials are thus currently underway to assess the efficacy of drugs. These include, for example, anti-interleukin 6 agents such as tocilizumab or sarilumab, antiviral agents such as remdesivir or the combination of lopinavir/ritonavir, and hydroxychloroquine. However, a therapeutic agent with a proven efficacy for preventing and/or treating COVID-19, in particular severe forms of COVID-19, is yet to be identified.

Therefore, there is still a need for effective treatment and prevention of diseases caused by a coronavirus, such as COVID-19, in particular to prevent the onset of severe forms of such diseases.

The present invention relates to the use of prostaglandin receptor agonists, especially prostaglandin receptor agonists that increase cAMP levels, such as EP2 and/or EP4 agonists, preferably EP2 agonists, in the treatment of a disease caused by a coronavirus, such as COVID-19, in a subject in need thereof, especially in subjects at risk to develop a severe form and/or a complication of a disease caused by a coronavirus.

### SUMMARY

This invention thus relates EP2 and/or EP4 agonists for use in the treatment of a disease caused by a coronavirus infection in a subject in need thereof.

In one embodiment, the EP2 and/or EP4 agonists are selected from prostaglandin E1 (PGE1), prostaglandin E2 (PGE2), butaprost, butaprost free acid, ONO-AE1-259-01, taprenepag, taprenepag isopropyl, evatanepag, PGN-9856, omidenepag, 19-hydroxy-PGE2, 1-OH-PGE1, 11-deoxy-PGE2, 13,14-dihydro-PGE1, L902688, CP734432, TCS 2510 and ONO-AE1-437. In a specific embodiment, the agonist is an EP2 agonist selected from prostaglandin E1 (PGE1), prostaglandin E2 (PGE2), butaprost, butaprost free acid, ONO-AE1-259-01, taprenepag, taprenepag isopropyl, evatanepag, PGN-9856, omidenepag, 19-hydroxy-PGE2. In a preferred embodiment, the agonist is prostaglandin E1 (PGE1), taprenepag or taprenepag isopropyl.

In one embodiment, the coronavirus is selected from HCoV-229E, HCoV-NL63, HCoV-OC43, HCoV-HKU1, MERS-CoV, SARS-CoV-1 and SARS-CoV-2; preferably the coronavirus is selected from MERS-CoV, SARS-CoV-1 and SARS-CoV-2. In a specific embodiment, the coronavirus is SARS-CoV-2 and the disease caused by the coronavirus infection is coronavirus disease 2019 (COVID-19).

In one embodiment, the subject is infected by SARS-CoV-2 from less than 10 days, preferably from less than 8 days, more preferably from less than 6 days. In one embodiment, the subject suffers from a mild or moderate form of COVID-19. In one embodiment, the subject is at risk to develop a severe form and/or a complication of COVID-19. In one embodiment, a severe form and/or complication of COVID-19 is selected from respiratory failure, including acute respiratory failure or acute respiratory distress syndrome (ARDS); persistence of respiratory failure including the requirement for prolonged mechanical ventilation, in particular prolonged mechanical ventilation lasting more than 15 days, and failed extubation; secondary infection or superinfection; thrombotic complications including venous and/or arterial thromboembolism; pulmonary embolism; cardiocirculatory failure (which may also be referred to as cardiovascular failure); renal failure including acute kidney injury (AKI); liver failure; and any combinations thereof.

In one embodiment, the subject present one or more of the following risk factors:
- the subject is older than 60, 65, 70, 75, 80 or 85 years of age;
- the subject suffers from at least one comorbidity selected from acute kidney injury, asthma, atopy, autoimmune or auto-inflammatory diseases or conditions, bone marrow or stem cell transplantations in the past 6 months, bronchial hyperreactivity, cardiovascular diseases or conditions, chronic bronchitis, chronic kidney diseases, chronic liver disease, chronic obstructive pulmonary disease (COPD), ciliary deficiencies (hereditary or acute ciliary deficiencies), cystic fibrosis, diabetes, emphysema, hematological diseases, high blood pressure, immunodeficiency, infection with HIV, malignancy *(i.e.,* cancer), obesity, pulmonary hypertension, rare diseases and inborn errors of metabolism that significantly increase the risk of infections (such as severe combined immunodeficiency), reactive airway disease, recipient of solid organ transplants, and severe respiratory conditions;
- the subject receives or has recently received one or more of the treatments selected from active chemotherapy or radical radiotherapy for lung cancer, immunosuppression therapy in particular immunosuppression therapy sufficient to significantly increase the risk of infection, immunotherapy or antibody treatment for cancer, and targeted cancer treatments that can affect the immune system (such as protein kinase inhibitors or PARP inhibitors);
- the subject has one or more of the habits or behaviors selected from active smoking, chronic passive smoking (also referred to as environmental exposure smoking).

In one embodiment, the subject presents low early IFN-gamma response.

In one embodiment, the EP2 and/or EP4 agonist is to be administered simultaneously, separately or sequentially with at least one further pharmaceutically active agent selected from anti-viral agents, anti-interleukin 6 (anti-IL-6) agents, other agents such as chloroquine or hydroxychloroquine, and any mixtures thereof.

The present invention also relates to a method for identifying a subject suffering from a disease caused by a coronavirus infection, preferably from a mild to moderate form of COVID-19, susceptible to respond to an EP2 and/or EP4 agonist, said method comprising:
- measuring the level of expression of ACE2 in a biological sample from the subject; and
- comparing the level of expression of ACE2 measured in the biological sample from the subject to a reference value.

The present invention further provides a method for identifying a subject suffering from a disease caused by a coronavirus infection, preferably from a mild to moderate form of COVID-19, susceptible to respond to an EP2 and/or EP4 agonist, said method comprising:
- measuring the level of expression of one or more of IFIT1, IFIT2 and IFIT3 genes in a biological sample from the subject; and
- comparing the level of expression of one or more of IFIT1, IFIT2 and IFIT3 genes measured in the biological sample from the subject to a reference value.

The invention also provides an EP2 and/or EP4 agonist for use in a method to regulate interferon signaling pathway in a subject in need thereof; preferably to induce one or more of IFIT1, IFIT2 and IFIT3 genes in a subject in need thereof.

### DEFINITIONS

In the present invention, the following terms have the following meanings:
- **"Agonist"** refers to a natural or synthetic compound which activates a biological response when it binds to a receptor. Therefore, a **"prostaglandin receptor agonist"** includes any compound that, upon administration to a subject, result in stimulation of a biological response associated with activation of at least one prostaglandin receptor in the subject, including any of the downstream biological effects otherwise resulting from the binding to the prostaglandin receptor(s) of its natural ligand(s). In one embodiment, the prostaglandin receptor is EP2 receptor and/or EP4 receptor.
- **"EP2 and/or EP4 agonist"** refers to an EP2 agonist, an EP4 agonist or an EP2/EP4 agonist. **"EP2 agonist"** refers to an agonist of EP2 receptor. **"EP4 agonist"** refers to an agonist of EP4 receptor. "**EP2/EP4 agonist"** refers to an agonist of both EP2 and EP4 receptors.
- **"Disease caused by a coronavirus"** and **"disease caused by a coronavirus infection"** are interchangeable and refer to any symptom or set of symptoms induced in a subject by the presence of a coronavirus in the organism of said subject.
- **"Pharmaceutically acceptable excipient"** or **"pharmaceutically acceptable carrier"** refers to an excipient or carrier that does not produce an adverse, allergic or other untoward reaction when administered to a mammal, preferably a human. This includes any and all solvents, dispersion media, coatings, antibacterial and antifungal agents, isotonic agents, absorption delaying agents and other similar ingredients. For human administration, preparations should meet sterility, pyrogenicity, general safety and purity standards as required by the regulatory offices such as the FDA (US Food and Drug Administration) or EMA (European Medicines Agency).
- **"Respiratory support"** refers to any measure administered to a subject in order to compensate for a respiratory distress or failure experienced by the subject. Examples of such measures include non-invasive ventilation (NIV) such as supplemental oxygen (also called oxygen therapy) by mask or nasal prongs, positive pressure, high flow nasal oxygen; invasive mechanical ventilation (IMV) requiring tracheal intubation or tracheostomy; and extracorporeal membrane oxygenation (ECMO). As used herein, "respiratory support" or "oxygen therapy" thus encompass both non-invasive ventilation (NIV) and invasive mechanical ventilation (IMV).
- **"Subject"** refers to a mammal, preferably a human. According to the present invention, a subject is a mammal, preferably a human, suffering from a disease caused by a coronavirus, in particular from COVID-19 caused by a SARS-CoV-2. In one embodiment, the subject is a "patient", *i.e*., a mammal, preferably a human, who/which is awaiting the receipt of, or is receiving medical care or was/is/will be the object of a medical procedure, or is monitored for the development of disease caused by a coronavirus, in particular COVID-19 caused by a SARS-CoV-2.
- **"Therapeutically effective amount"** or **"therapeutically effective dose"** refers to the amount or dose or concentration of a prostaglandin receptor agonist as described herein that is aimed at, without causing significant negative or adverse side effects to the subject in need of treatment, preventing, reducing, alleviating or slowing down (lessening) one or more of the symptoms or manifestations of a disease caused by a coronavirus, in particular COVID-19 caused by a SARS-CoV-2.
- **"Treating"** or **"Treatment"** refers to a therapeutic treatment, to a prophylactic (or preventative) treatment, or to both a therapeutic treatment and a prophylactic (or preventative) treatment, wherein the object is to prevent, reduce, alleviate, and/or slow down (lessen) one or more of the symptoms or manifestations of a disease caused by a coronavirus, in particular COVID-19 caused by a SARS-CoV-2, in a subject in need thereof. Symptoms of a disease caused by a coronavirus, in particular COVID-19 caused by a SARS-CoV-2, include, without being limited to, headache, muscle pain, fatigue, fever, anosmia, ageusia, and respiratory symptoms such as dry cough and/or breathing difficulties that may require respiratory support (for example supplemental oxygen, non-invasive ventilation, invasive mechanical ventilation, extracorporeal membrane oxygenation (ECMO)). Manifestations of a disease caused by a coronavirus, in particular COVID-19 caused by a SARS-CoV-2, also include, without being limited to, the viral load (also known as viral burden or viral titer) detected in a biological sample from the subject. In one embodiment, "treating" or "treatment" refers to a therapeutic treatment. In another embodiment, "treating" or "treatment" refers to a prophylactic or preventive treatment. In yet another embodiment, "treating" or "treatment" refers to both a prophylactic (or preventive) treatment and a therapeutic treatment. In one embodiment, the object of the treatment according to the present invention is to bring about at least one of the following:
   ∘ an improvement in the clinical status, for example defined as a decrease in the score assessed according with an ordinal scale such as the 8-point ordinal scale as defined hereinafter;
   ∘ a decrease in the requirement for respiratory support;
   ∘ a decrease in the requirement for other organ support, such as cardiovascular support and/or renal replacement therapy;
   ∘ a discharge from the intensive care unit;
   ∘ a discharge from hospital; and/or
   ∘ a reduction in the viral load detected in a sample from the subject.
- "**8-point ordinal scale**" as used herein refers to a tool for assessing the clinical status of a subject suffering from a disease caused by a coronavirus, in particular COVID-19 caused by a SARS-CoV-2. The 8-point ordinal scale ranges from 1 to 8, with a lower score corresponding to a better clinical status as indicated below:
   ∘ a score of 1 corresponds to a subject not hospitalized, with no limitations on activities;
   ∘ a score of 2 corresponds to a subject not hospitalized, with limitations on activities;
   ∘ a score of 3 corresponds to a subject hospitalized, not requiring supplemental oxygen;
   ∘ a score of 4 corresponds to a subject hospitalized, requiring supplemental oxygen by mask or nasal prongs;
   ∘ a score of 5 corresponds to a subject hospitalized, on non-invasive ventilation or high flow oxygen devices;
   ∘ a score of 6 corresponds to a subject hospitalized, on intubation and mechanical ventilation;
   ∘ a score of 7 corresponds to a subject hospitalized, on invasive mechanical ventilation and additional organ support such as pressors, renal replacement therapy (RRT) and extracorporeal membrane oxygenation (ECMO);
   ∘ a score of 8 corresponds to death.

### DETAILED DESCRIPTION

The present invention thus relates to the treatment of a disease caused by a coronavirus infection, such as COVID-19, in a subject in need thereof, using prostaglandin receptor agonists, especially prostaglandin receptor agonists that increase cAMP levels, such as EP2 and/or EP4 agonists, more preferably EP2 agonists.

### Prostaglandin receptor agonists increasing cAMP

Prostaglandin receptors or prostanoid receptors represent a sub-class of cell surface membrane receptors that are regarded as the primary receptors for one or more of the naturally occurring prostanoids. When activated, some of the prostaglandin receptors increase the production of cyclic adenosine monophosphate (cAMP): this is the case of prostaglandin EP2 receptor (EP2), prostaglandin EP4 receptor (EP4), prostaglandin DP1 receptor (DP1) and prostacyclin I2 receptor (IP).

Cyclic AMP is a second messenger important in many biological processes, involved in intracellular signal transduction, in cAMP-dependent pathways. It is especially involved in the activation of protein kinases and thereby plays a role in inflammation mechanisms.

In one embodiment, the invention thus relates to the treatment of a disease caused by a coronavirus infection, such as COVID-19, in a subject in need thereof, using prostaglandin receptor agonists that increase cAMP levels. Preferably, prostaglandin receptor agonists are selected from EP2 agonists, EP4 agonists and EP2/EP4 agonists. By "EP2/EP4 agonists" it is referred to compounds that are agonists of both EP2 and EP4 receptors.

In one embodiment, the invention thus relates to the treatment of a disease caused by a coronavirus infection, such as COVID-19, in a subject in need thereof, using EP2 and/or EP4 agonists, i.e. agonists of EP2 receptor, agonists of EP4 receptor or agonists of both EP2 and EP4 receptors. In a preferred embodiment, the invention relates to the treatment of a disease caused by a coronavirus infection, such as COVID-19, in a subject in need thereof, using EP2 agonists.

Prostaglandin E2 receptor 2, also known as EP2, is a G protein-coupled receptor (GPCR) for prostaglandin E2 (PGE2). It is one of four identified EP receptors, the others being EP1, EP3, and EP4, which bind with and mediate cellular responses to PGE2 and also, but with lesser affinity and responsiveness, certain other prostanoids. The EP2 receptor is widely distributed in humans. EP2 is implicated in various physiological and pathological responses.

In one embodiment, the EP2 agonist is selected from prostaglandin E1 (PGE1), prostaglandin E2 (PGE2), butaprost, butaprost free acid, ONO-AE1-259-01, taprenepag, taprenepag isopropyl, evatanepag, PGN-9856, omidenepag and 19-hydroxy-PGE2. Preferably, the EP2 agonist is selected from prostaglandin E1 (PGE1), taprenepag and taprenepag isopropyl. In one embodiment, the EP2 agonist is prostaglandin E1 (PGE1). In one embodiment, the EP2 agonist is taprenepag or taprenepag isopropyl. In one embodiment, the EP2 agonist is taprenepag. In one embodiment, the EP2 agonist is taprenepag isopropyl.

Prostaglandin E1 (PGE1), also known as alprostadil, has the following chemical structure:

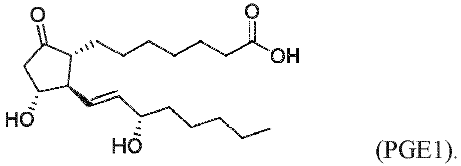

Prostaglandin E2 (PGE2), also known as dinoprostone, has the following chemical structure:

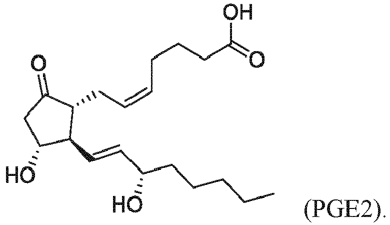

Butaprost and butaprost free acid have the following chemical structures:

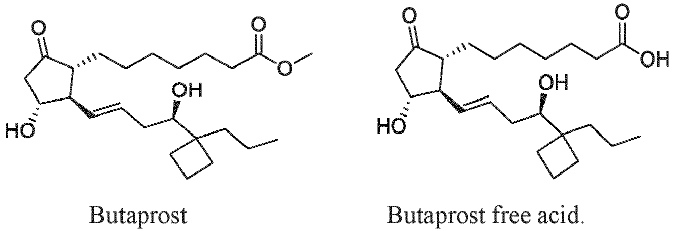

ONO-AE1-259-01 has the following chemical structure:

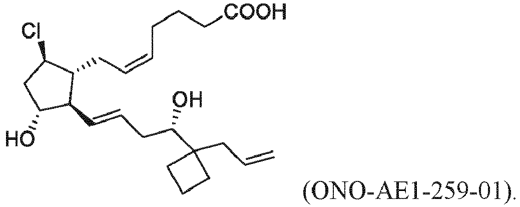

Taprenepag, also known as CP 544326, and its isopropyl prodrug taprenepag isopropyl (PF04217329) have the following chemical structures:

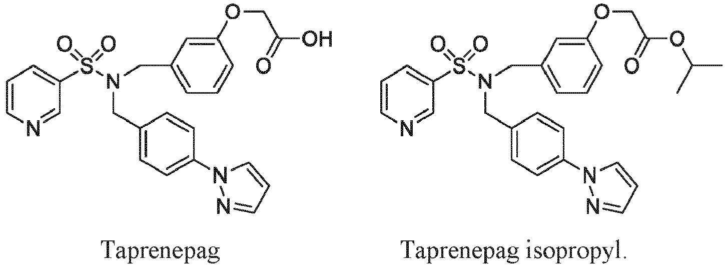

Evatanepag, also known as CP-533536, has the following chemical structure:

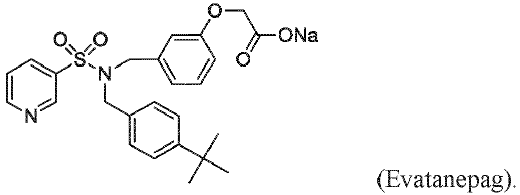

PGN-9856 has the following chemical structure:

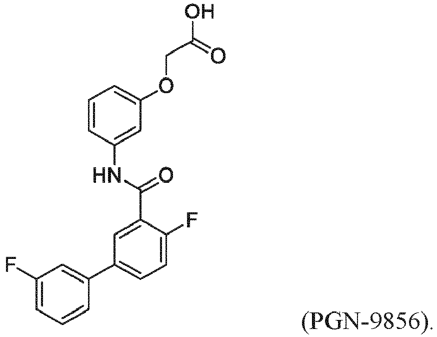

Omidenepag has the following chemical structure:

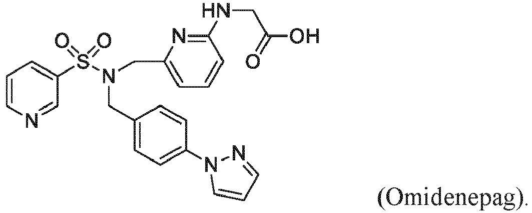

19-hydroxy-PGE2 has the following chemical structure:

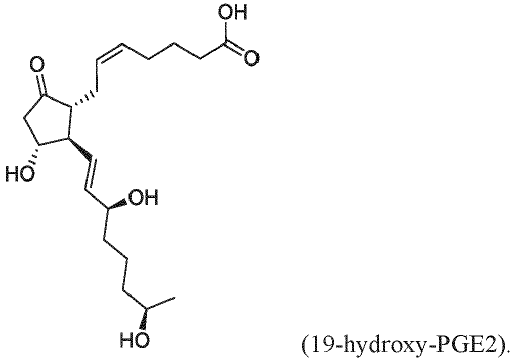

In one embodiment, the EP2 agonist can also be an agonist of one or more of EP1, EP3 and EP4. In one embodiment, the EP2 agonist is also an EP4 agonist. For example, prostaglandin E1 (PGE1) is an EP1, EP2, EP3 and EP4 agonist.

Prostaglandin E4 receptor 4, also known as EP4, is a G protein-coupled receptor (GPCR) for prostaglandin E2 (PGE2). It binds with and mediate cellular responses to PGE2 and also, but with lesser affinity and responsiveness, certain other prostanoids. The EP4 receptor is implicated in various physiological and pathological responses.

In one embodiment, the EP4 agonist is selected from prostaglandin E1 (PGE1), derivatives thereof and non prostanoid like agonists. Preferably, the EP4 agonist is selected from prostaglandin E1 (PGE1), 1-OH-PGE1, 11-deoxy-PGE2, 13,14-dihydro-PGE1, L902688, CP734432, TCS 2510 and ONO-AE1-437. In one embodiment, the EP4 agonist is prostaglandin E1 (PGE1).

1-OH-PGE1, also known as 19-hydroxy-PGE1, has the following chemical structure:

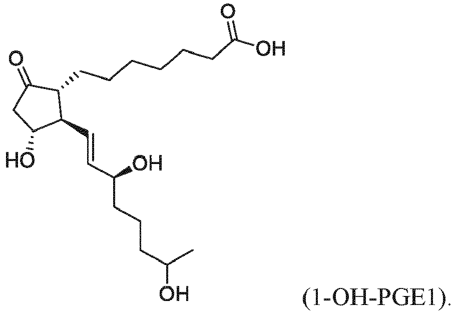

11-deoxy-PGE2 has the following chemical structure:

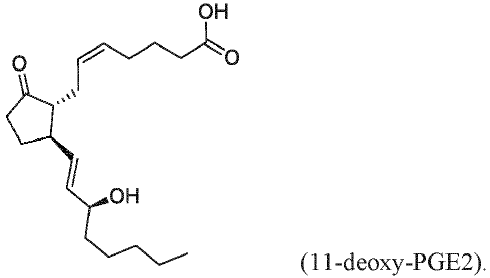

13,14-dihydro-PGE1 has the following chemical structure:

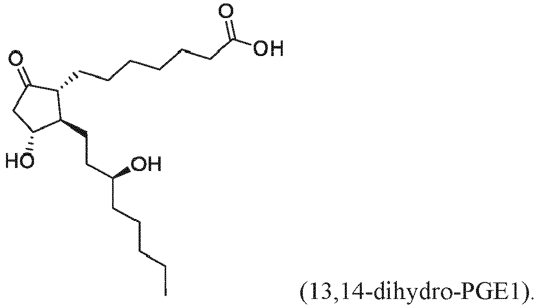

L902688, also known as UNII-D17QSK5F4B, has the following chemical structure:

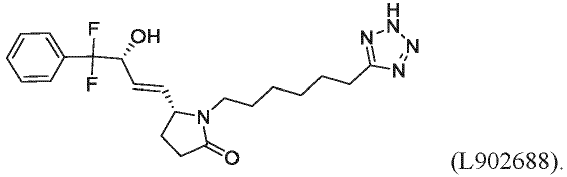

CP734432, also known as CID 73755071, has the following chemical structure:

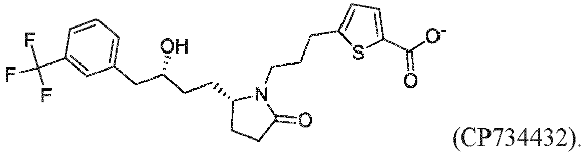

TCS 2510 has the following chemical structure:

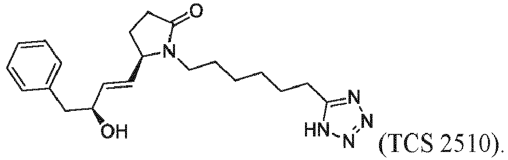

ONO-AE1-437 has the following chemical structure:

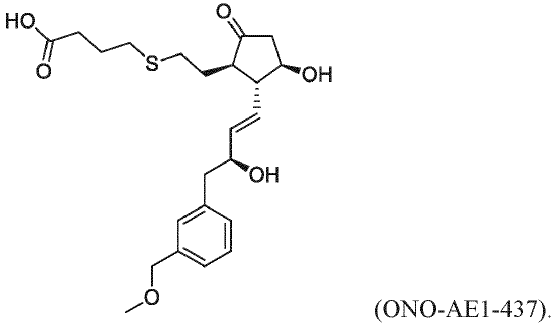

In one embodiment, the EP4 agonist can also be an agonist of one or more of EP1, EP2 and EP3. In one embodiment, the EP4 agonist is also an EP2 agonist. For example, prostaglandin E1 (PGE1) is an EP1, EP2, EP3 and EP4 agonist.

In one embodiment, prostaglandin receptor agonists useful according to the invention are selected from: prostaglandin E1 (PGE1), prostaglandin E2 (PGE2), butaprost, butaprost free acid, ONO-AE1-259-01, taprenepag, taprenepag isopropyl, evatanepag, PGN-9856, omidenepag, 19-hydroxy-PGE2, 1-OH-PGE1, 11-deoxy-PGE2, 13,14-dihydro-PGE1, L902688, CP734432, TCS 2510 and ONO-AE1-437.

### Disease

This invention thus relates to a prostaglandin receptor agonist for use in the treatment of a disease caused by a coronavirus infection, the prostaglandin receptor agonist increasing cAMP levels, such as for example an EP2 and/or EP4 agonist, preferably an EP2 agonist.

In one embodiment, the coronavirus is a human coronavirus. In one embodiment, the coronavirus is an alpha coronavirus or a beta coronavirus, preferably a beta coronavirus.

Examples of alpha coronaviruses include, without being limited to, human coronavirus 229E (HCoV-229E) and human coronavirus NL63 (HCoV-NL63) also sometimes known as HCoV-NH or New Haven human coronavirus.

Examples of beta coronaviruses include, without being limited to, human coronavirus OC43 (HCoV-OC43), human coronavirus HKU1 (HCoV-HKU1), Middle East respiratory syndrome-related coronavirus (MERS-CoV) previously known as novel coronavirus 2012 or HCoV-EMC, severe acute respiratory syndrome coronavirus (SARS-CoV) also known as SARS-CoV-1 or SARS-classic, and severe acute respiratory syndrome coronavirus (SARS-CoV-2) also known as 2019-nCoV or novel coronavirus 2019.

In one embodiment, the coronavirus is selected from the group comprising or consisting of HCoV-229E, HCoV-NL63, HCoV-OC43, HCoV-HKU1, MERS-CoV, SARS-CoV-1 and SARS-CoV-2. In one embodiment, the coronavirus is selected from the group comprising or consisting of MERS-CoV, SARS-CoV-1 and SARS-CoV-2.

In one embodiment, the coronavirus is a MERS coronavirus, in particular MERS-CoV causing Middle East respiratory syndrome (MERS). Thus, in one embodiment, the subject is suffering from MERS caused by MERS-CoV.

In one embodiment, the coronavirus is a SARS coronavirus. In one embodiment, the coronavirus is SARS-CoV (also referred to as SARS-CoV-1) causing severe acute respiratory syndrome (SARS) or SARS-CoV-2 causing COVID-19. Thus, in one embodiment, the subject is suffering from SARS caused by SARS-CoV-1 or from COVID-19 caused by SARS-CoV-2. In one embodiment, the coronavirus is SARS-CoV-2 causing COVID-19. Thus, in one in one embodiment, the subject is suffering from COVID-19 caused by SARS-CoV 2.

Thus, in one embodiment, prostaglandin receptor agonists, preferably EP2 and/or EP4 agonists, are for use in the treatment of a coronavirus infection selected from MERS-CoV, SARS-CoV-1 and SARS-CoV-2. In one embodiment, prostaglandin receptor agonists, preferably EP2 and/or EP4 agonists, are for use in the treatment of MERS, SARS and COVID-19. According to a preferred embodiment, prostaglandin receptor agonists, preferably EP2 and/or EP4 agonists, are for use in the treatment of COVID-19.

As mentioned above "disease caused by a coronavirus" or "disease caused by a coronavirus infection" are interchangeable and refer to any symptom or set of symptoms induced in a subject by the presence of a coronavirus in the organism of said subject.

Diseases caused by a coronavirus, especially COVID-19 caused by a SARS-CoV-2, have a broad clinical spectrum, from mild or moderate symptoms to more severe forms, with a wide variety of complications.

Especially, subjects affected by COVID-19 generally present first with symptoms including headache, muscle pain, fatigue, fever and respiratory symptoms such as dry cough and/or breathing difficulties that may require respiratory support (for example supplemental oxygen, non-invasive ventilation, invasive mechanical ventilation, extracorporeal membrane oxygenation (ECMO)). Other reported symptoms include anosmia and/or ageusia.

Some subjects develop a severe form of COVID-19 that may lead to respiratory complications such as pneumonitis and acute respiratory failure. Complications of COVID-19 also include extra-respiratory complications such as thrombotic complications, pulmonary embolism, cardiovascular failure, renal failure, liver failure and secondary infections.

In one embodiment, the severity of the disease caused by a coronavirus, especially COVID-19 caused by a SARS-CoV-2, can be evaluated by the World Health Organization (WHO) COVID ordinal scale for clinical improvement. This WHO COVID ordinal scale provides a score ranging from 0 to 8 depending on the patient's state, as shown in Table 1 hereafter.

**Table 1: WHO COVID ordinal scale**

| **Patient State** | **Descriptor** | **Score** |
|---|---|---|
| Uninfected | No clinical or virological evidence of infection | 0 |
| Ambulatory Mild disease | No limitation of activities | 1 |
| | Limitation of activities | 2 |
| Hospitalized Moderate disease | Hospitalized, no oxygen therapy | 3 |
| | Oxygen by mask or nasal prongs | 4 |
| Hospitalized Severe disease | Non-invasive ventilation or high-flow oxygen | 5 |
| | Intubation and mechanical ventilation | 6 |
| | Ventilation + additional organ support - pressors, renal replacement therapy (RRT), extracorporeal membrane oxygenation (ECMO) | 7 |
| Dead | Death | 8 |

In one embodiment, a mild form of COVID-19 corresponding to a score of 1 or 2 according to the WHO COVID ordinal scale. In one embodiment, a moderate form of COVID-19 corresponding to a score of 3 or 4 according to the WHO COVID ordinal scale. In one embodiment, a severe form of COVID-19 corresponds to a score ranging from 5 to 7 according to the WHO COVID ordinal scale.

In one embodiment, the severity of COVID-19 caused by a SARS-CoV-2, can also be evaluated according to the WHO criteria of severity as follows:
- mild: cases showing mild clinical symptoms without evidence of viral pneumonia or hypoxia.
- moderate: cases showing fever and respiratory symptoms (such as a cough, shortness of breath, and/or chest tightness) with radiological findings of pneumonia and that may require (O₂): 3L/min < oxygen < 5L/min
- severe: cases meeting any of the following criteria:
   - respiratory distress (respiratory rate (RR) ≧ 30 breaths/ min);
   - oxygen saturation (SpO₂) ≤ 93% at rest in ambient air; or SpO₂ ≤ 97% with O₂ > 5L/min;
   - ratio of artery partial pressure of oxygen/inspired oxygen fraction (PaO₂/FiO₂) ≦ 300 mmHg (1 mmHg = 0.133 kPa), PaO₂/FiO₂ in high-altitude areas (at an altitude of over 1,000 meters above the sea level) shall be corrected by the following formula: PaO₂/FiO₂ [multiplied by] [Atmospheric pressure (mmHg)/760]; and/or
   - chest imaging that showed obvious lesion progression within 24-48 hours > 50%.
- critical: cases meeting any of the following criteria:
   - respiratory failure and requiring mechanical ventilation;
   - shock; and/or
   - multiple organ failure (extra pulmonary organ failure) requiring admission to intensive care unit (ICU).

In one embodiment, a severe form of the disease caused by a coronavirus, in particular a severe form COVID-19, is defined as requiring hospitalization. In one embodiment, a severe form of the disease caused by a coronavirus, in particular a severe form COVID-19, is defined as requiring admission in intensive care unit (ICU).

In one embodiment, a severe form of the disease caused by a coronavirus, in particular a severe form COVID-19, is defined as requiring respiratory support as defined hereinabove. In one embodiment, the respiratory support is selected from the group comprising or consisting of non-invasive ventilation (NIV) such as supplemental oxygen (also called oxygen therapy) by mask or nasal prongs, positive pressure, high flow nasal oxygen; invasive mechanical ventilation (IMV) requiring tracheal intubation or tracheostomy; and extracorporeal membrane oxygenation (ECMO). In one embodiment, a severe form of the disease caused by a coronavirus, in particular a severe form COVID-19, is defined as requiring invasive mechanical ventilation as described hereinabove.

In one embodiment, the complication of the disease caused by a coronavirus, in particular of COVID-19, is selected from the group comprising or consisting of, respiratory failure, including acute respiratory failure or acute respiratory distress syndrome (ARDS); persistence of respiratory failure including the requirement for prolonged mechanical ventilation, in particular prolonged mechanical ventilation lasting more than 15 days, and failed extubation; secondary infection or superinfection; thrombotic complications including venous and/or arterial thromboembolism; pulmonary embolism; cardiocirculatory failure (which may also be referred to as cardiovascular failure); renal failure including acute kidney injury (AKI); liver failure; and any combinations thereof.

### Subject

In one embodiment, the subject is a male. In one embodiment, the subject is a female.

In a preferred embodiment the subject is an adult. Thus, in one embodiment, the subject is older than 18, 19, 20 or 21 years of age. In another embodiment the subject is a child. Thus, in one embodiment, the subject is younger 18, 17, 16 or 15 years of age.

In one embodiment, the subject is younger than 85, 80, 75, 70, 65 or 60 years of age. In one embodiment, the subject is older than 60, 65, 70, 75, 80 or 85 years of age. In one embodiment, the subject is 60 years old or older. In one embodiment, the subject is 70 years old or older. In one embodiment, the subject is 75 years old or older. In one embodiment, the subject is 80 years old or older.

In one embodiment, the subject does not suffer from any underlying condition or disease.

In another embodiment, the subject suffers from at least one comorbidity. As used herein, "comorbidity" refers to a disease or condition coexisting in the subject with the disease caused by a coronavirus.

Examples of comorbidities that may coexist in the subject with a disease caused by a coronavirus such as a SARS-CoV-2 infection causing COVID-19, include, without being limited to, acute kidney injury, asthma, atopy, autoimmune or auto-inflammatory diseases or conditions, bone marrow or stem cell transplantations in the past 6 months, bronchial hyperreactivity, cardiovascular diseases or conditions, chronic bronchitis, chronic kidney diseases, chronic liver disease, chronic obstructive pulmonary disease (COPD), ciliary deficiencies (hereditary or acute ciliary deficiencies), cystic fibrosis, diabetes, emphysema, hematological diseases, high blood pressure, immunodeficiency, infection with HIV, malignancy (*i.e.,* cancer), obesity, pulmonary hypertension, rare diseases and inborn errors of metabolism that significantly increase the risk of infections (such as severe combined immunodeficiency), reactive airway disease, recipient of solid organ transplants, and severe respiratory conditions.

In one embodiment, the subject presents at least one comorbidity selected from the group comprising or consisting of acute kidney injury, asthma, atopy, autoimmune or auto-inflammatory diseases or conditions, bone marrow or stem cell transplantations in the past 6 months, bronchial hyperreactivity, cardiovascular diseases or conditions, chronic bronchitis, chronic kidney diseases, chronic liver disease, chronic obstructive pulmonary disease (COPD), ciliary deficiencies (hereditary or acute ciliary deficiencies), cystic fibrosis, diabetes, emphysema, hematological diseases, high blood pressure, immunodeficiency, infection with HIV, malignancy (*i.e*., cancer), obesity, pulmonary hypertension, rare diseases and inborn errors of metabolism that significantly increase the risk of infections (such as severe combined immunodeficiency), reactive airway disease, recipient of solid organ transplants, and severe respiratory conditions.

In one embodiment, the subject presents at least one disease selected from ciliary deficiencies (hereditary or acute ciliary deficiencies) and immunodeficiencies.

Examples of ciliary deficiencies include nephronophthisis (NPHP), Senior Loken syndrome (SLS), Joubert syndrome (JBTS), Bardet Biedl syndrome (BBS), Meckel Gruber syndrome (MKS), orofacialdigital syndrome (OFD), Jeune syndrome (JATD) and deficiencies of motile cilia such as primary ciliary dyskinesia (PCD).

In one embodiment, the subject is diagnosed with a SARS-CoV-2 infection. Methods for diagnosing a SARS-CoV-2 infection include, but are not limited to, rRT-PCR (real-time reverse transcription polymerase chain reaction) test allowing to detect the presence of SARS-CoV-2 in a sample from a subject (such as a sample from a nasal swab, a sample from an oropharyngeal swab, a sputum sample, a lower respiratory tract aspirate, a bronchoalveolar lavage, a nasopharyngeal wash/aspirate or a nasal aspirate) or an antibody test (such as an enzyme-linked immunosorbent assay (ELISA)) allowing to detect the presence of antibodies against SARS-CoV-2 in a sample from a subject (such as a blood sample).

In one embodiment, the subject is infected by SARS-CoV-2 from less than 10 days, preferably from less than 8 days, more preferably from less than 6 days.

In one embodiment, the subject presents at least one of the following symptoms: cough, shortness of breath or difficulty breathing.

In one embodiment, the subject presents at least one, preferably at least two, of the following symptoms: fever (*i.e*., any body temperature over 38°C), chills, repeated shaking with chills, muscle pain, headache, sore throat and new loss of taste or smell.

In one embodiment, the subject is suffering from a mild form of COVID-19, corresponding to a score of 1 or 2 according to the WHO COVID scoring scale (according to Table 1 above). In one embodiment, the subject is suffering from a moderate form of COVID-19, corresponding to a score of 3 or 4 according to the WHO COVID scoring scale. In another embodiment, the subject is suffering from a severe form of COVID-19 corresponding to a score ranging from 5 to 7 according to the WHO COVID scoring scale. In a preferred embodiment, the use of prostaglandin receptor agonists, preferably EP2 and/or EP4 agonists, according to the invention is particularly useful for subjects suffering from a mild or moderate form of COVID-19.

In one embodiment, the subject is not hospitalized. In one embodiment, the subject is hospitalized.

In one embodiment, the subject is hospitalized but does not require admission in ICU. In one embodiment, the subject is hospitalized and requires admission in ICU. In one embodiment, the subject is hospitalized in ICU.

In one embodiment, the subject is hospitalized and does not require respiratory support.

In one embodiment, the subject is hospitalized and requires respiratory support. In one embodiment, the subject is hospitalized and requires non-invasive ventilation (NIV). In one embodiment, the subject is hospitalized in ICU and requires invasive mechanical ventilation. In one embodiment, the subject is hospitalized in ICU and is under invasive mechanical ventilation. In one embodiment, the subject is hospitalized in ICU and has been under invasive mechanical ventilation for less than 6 hours, 12 hours, 18 hours, 24 hours, 30 hours, 36 hours, 42 hours, or 48 hours, preferably for less than 48 hours.

### Risk factors

In one embodiment, prostaglandin receptor agonists, preferably EP2 and/or EP4 agonists, as herein defined are especially useful for the treatment of a subject who is at risk of developing a severe form and/or a complication of a disease caused by a coronavirus infection, especially a severe form and/or a complication of COVID-19. Severe forms and complications of COVID-19 are described above.

In one embodiment, the subject is suffering from a disease caused by a coronavirus infection, such as COVID-19, and is at risk of developing a severe form and/or a complication of a disease caused by a coronavirus infection, especially a severe form and/or a complication of COVID-19. Preferably, the subject is suffering from a mild or moderate form of COVID-19 as defined above, and is at risk of developing a severe form and/or a complication of COVID-19.

In one embodiment, the subject is at risk of developing a severe form and/or a complication of a disease caused by a coronavirus infection, especially a severe form and/or a complication of COVID-19, when having at least one risk factor *i.e.* a preexisting disease, condition, habit or behavior that may lead to an increased risk of developing a severe form and/or a complication of a disease caused by a coronavirus infection, especially a severe form and/or a complication of COVID-19.

In one embodiment, the subject present one or more of the following risk factors:
- the subject is older than 60, 65, 70, 75, 80 or 85 years of age;
- the subject suffers from at least one comorbidity, as defined above, and such as acute kidney injury, asthma, atopy, autoimmune or auto-inflammatory diseases or conditions, bone marrow or stem cell transplantations in the past 6 months, bronchial hyperreactivity, cardiovascular diseases or conditions, chronic bronchitis, chronic kidney diseases, chronic liver disease, chronic obstructive pulmonary disease (COPD), ciliary deficiencies (hereditary or acute ciliary deficiencies), cystic fibrosis, diabetes, emphysema, hematological diseases, high blood pressure, immunodeficiency, infection with HIV, malignancy (*i.e*., cancer), obesity, pulmonary hypertension, rare diseases and inborn errors of metabolism that significantly increase the risk of infections (such as severe combined immunodeficiency), reactive airway disease, recipient of solid organ transplants, and severe respiratory conditions;

- the subject receives or has recently received one or more of the treatments selected from active chemotherapy or radical radiotherapy for lung cancer, immunosuppression therapy in particular immunosuppression therapy sufficient to significantly increase the risk of infection, immunotherapy or antibody treatment for cancer, and targeted cancer treatments that can affect the immune system (such as protein kinase inhibitors or PARP inhibitors);
- the subject has one or more of the habits or behaviors selected from active smoking, chronic passive smoking (also referred to as environmental exposure smoking).

In one embodiment, the present invention relates to a prostaglandin receptor agonist, preferably a prostaglandin receptor agonist increasing cAMP, more preferably an EP2 and/or EP4 agonist, even more preferably an EP2 agonist, for use in the treatment of a disease caused by a coronavirus infection as described hereinabove, in particular COVID-19, in a subject in need thereof, wherein said subject is suffering from an mild to moderate form of said disease and at risk to develop a severe form and/or a complication of said disease. In one embodiment, the present invention relates to a prostaglandin receptor agonist, preferably a prostaglandin receptor agonist increasing cAMP, more preferably an EP2 and/or EP4 agonist, even more preferably an EP2 agonist, for use in the treatment of COVID-19, in a subject in need thereof, wherein said subject is suffering from an mild to moderate form of COVID-19 and at risk to develop a severe form and/or a complication form of COVID-19.

### Downregulation of ACE2 expression

ACE2 receptor appears to be used by SARS-CoV-2, as it is also the case for SARS-CoV-1, as receptor for viral cell entry. Without willing to be bound by a theory, it is thought that the efficacy of the prostaglandin receptor agonists, especially EP2 agonists, as reported in the present invention, might be due to a considerable reduction of viral entry, and especially limitation of its expansion at the vascular level, by downregulation of ACE2 expression. In the specific case of SARS-Cov-2, ACE2 seems to be not only a receptor to the virus, but might also contribute to post infection regulation, immune response, cytokine secretion, and viral genome replication. Reduction of ACE2 thus might also have additional benefit during the recovery phase of COVID-19.

In one embodiment, prostaglandin receptor agonists, especially EP2 agonists, reduce ACE2 expression and thus reduce viral entry.

In one embodiment, prostaglandin receptor agonists as herein defined, preferably EP2 agonists, are especially useful for the treatment of a subject suffering from COVID-19 and presenting high levels of expression of ACE2, especially high levels of expression of ACE2 in nasal epithelial cells.

The invention also relates to an *in vitro* method for identifying a subject suffering from a disease caused by a coronavirus infection, preferably from a mild to moderate form of COVID-19, susceptible to respond to a therapy by a prostaglandin receptor agonist, preferably an EP2 and/or EP4 agonist, said method comprising:
- measuring the level of expression of ACE2 in a biological sample from the subject; and
- comparing the level of expression of ACE2 measured in the biological sample from the subject to a reference value.

According to the method as described above, the level of ACE2 expression is measured in a biological sample from a subject suffering from a disease caused by a coronavirus infection, preferably from a mild to moderate form of COVID-19.

As used herein, "biological sample" refers to a biological sample isolated from a subject and can include, by way of example and not limitation, bodily fluids, cell samples and/or tissue extracts such as homogenates or solubilized tissue obtained from a subject.

In one embodiment, the present invention does not comprise obtaining a biological sample from a subject. Thus, in one embodiment, the biological sample from the subject is a biological sample previously obtained from the subject. Said biological sample may be conserved in adequate conditions before being used as described herein.

In one embodiment, the biological sample from the subject is a cell sample and/or tissue extract, preferably a nasal epithelial cells sample. Methods for obtaining a cell sample and/or tissue extract, especially a nasal epithelial cells sample, are routinely used in clinical laboratories.

According to the present invention, the level of expression of ACE2 may be measured by any known method in the art. Methods for measuring an expression level are well-known to the skilled artisan. For example, the level of expression of ACE2 can be evaluated by RT-PCR or SYBR green PCR analyses. Especially, the level of expression of ACE2 can be evaluated using the method used in Example 2.

According to one embodiment, the reference value is a reference level of expression of ACE2 derived from a reference population. In one embodiment, the reference value is derived from population studies, including, for example, subjects having a similar age range, or subjects in the same or similar ethnic group.

According to one embodiment, the reference value is derived from the measure of the level of expression of ACE2 in a biological sample obtained from one or more subjects who are substantially healthy. In one embodiment, a "substantially healthy subject" is a subject who has not been diagnosed or identified as having or suffering from a disease caused by a coronavirus. In one embodiment, a "substantially healthy subject" is a subject who has not been diagnosed or identified as having or suffering from a disease caused by a coronavirus or any other infection. Thus, in one embodiment, the reference value is a reference level of expression of ACE2, derived from a reference population of subjects who are substantially healthy.

According to another embodiment, the reference value is derived from the measure of the level of expression of ACE2 in a biological sample from one or more subjects diagnosed or identified as suffering, or having suffered, from a disease caused by a coronavirus, in particular from COVID-19 caused by SARS-CoV-2. Thus, in one embodiment, the reference value is a reference level of expression of ACE2 derived from a reference population of subjects diagnosed or identified as suffering, or having suffered, from a disease caused by a coronavirus, in particular from COVID-19 caused by SARS-CoV-2. In such case, the reference value can be derived from statistical analyses and/or risk prediction data of a reference population as described hereinabove obtained from mathematical algorithms and computed indices of a disease caused by a coronavirus, in particular COVID-19 caused by SARS-CoV-2.

In one embodiment, a level of expression of ACE2, measured in the biological sample from the subject, higher than the reference value as described hereinabove indicates that the subject as described hereinabove is susceptible to respond to a therapy by a prostaglandin receptor agonist, preferably an EP2 and/or EP4 agonist. By "higher" is it meant a level of expression of ACE2 which is at least 2 fold the reference value.

### IFN response

Some subjects suffering from COVID-19 present a low early IFN-gamma response. This IFN-gamma absence during the early stage response to COVID-19 infection leads to advanced viremia, and in the same time, triggers a massive cytokine storm counter reaction, and thus to severe forms of the disease.

In one embodiment, the prostaglandin receptor agonists, especially EP2 and/or EP4 agonists, enable to induce an interferon-like response, by inducing one or more of genes related to interferon signaling pathway. By "interferon-like response" it is referred to a biological response that is similar to the biological response that would have occurred in presence of standard levels of interferon.

In one embodiment, the invention provides the use of prostaglandin receptor agonists in a method to regulate interferon signaling pathway in a subject in need thereof, preferably to upregulate interferon signaling pathway. In one embodiment, the interferon signaling pathway is interferon alpha/beta signaling pathway, preferably R-HSA-909733 Reactome pathway. In another embodiment, the interferon signaling pathway is interferon gamma signaling pathway, preferably R-HSA-877300 Reactome pathway.

In one embodiment, the invention provides the use of prostaglandin receptor agonists in a method to induce one or more genes in a subject in need thereof, wherein the gene is selected from BIRC3, BST2, DDX58, DHX58, EIF2AK2, EIF4E3, GBP2//GBP7, GBP4, HLA-DRA, HERC5, IFI6, IFI27, IFI35, IFIH1, IFIT1, IFIT2, IFIT3, IFITM1, IRF7, ISG15, MIR3614//TRIM25, MX1, MX2, OASL, RSAD2, SAMHD1, STAT1, TNFSF12//TNFSF12-TNFSF13//TNFSF13, TNFSF13B, TRIM14, USP18, XAF1, and combination thereof. In a specific embodiment, the invention provides the use of prostaglandin receptor agonists in a method to induce one or more of IFIT1, IFIT2 and IFIT3 genes in a subject in need thereof.

Among the genes related to interferon pathway, prostaglandin receptor agonists, preferably EP2 agonists, especially upregulate the expression of interferon induced protein with tetratricopeptide repeats 1 (IFIT1), interferon induced protein with tetratricopeptide repeats 2 (IFIT2), and interferon induced protein with tetratricopeptide repeats 3 (IFIT3).

In one embodiment, the invention thus provides prostaglandin receptor agonists, especially EP2 and/or EP4 agonists for use in a method to induce one or more of IFIT1, IFIT2 and IFIT3 genes in a subject in need thereof.

By increasing the expression of INF post viral infection, prostaglandin receptor agonists, especially EP2 agonists, lead to a significant IFN release on immune cells involved in the innate response to infection by coronaviruses. Restoring the mediating molecules of innate immunity in the early stages of the infection more quickly trigger the adaptive immune response, necessary to control the progression of the viremia causing the explosive and deleterious cytokine response.

In one embodiment, prostaglandin receptor agonists as herein defined are thus especially useful for the treatment of a subject suffering from COVID-19 and presenting low early IFN-gamma response. In one embodiment, prostaglandin receptor agonists increasing cAMP, such as EP2 and/or EP4 agonists, as herein defined are also especially useful for the treatment of a subject suffering from COVID-19 and presenting low early IFN-gamma response. In one embodiment, a "low IFN-gamma response" corresponds to a downregulation by at least 2 fold of IFN-gamma response.

The invention also relates to an *in vitro* method for identifying a subject suffering from a disease caused by a coronavirus infection, preferably from a mild to moderate form of COVID-19, susceptible to respond to a therapy by a prostaglandin receptor agonist, preferably an EP2 and/or EP4 agonist, said method comprising:
- measuring the level of expression of one or more of IFIT1, IFIT2 and IFIT3 genes in a biological sample from the subject; and
- comparing the level of expression of one or more of IFIT1, IFIT2 and IFIT3 genes measured in the biological sample from the subject to a reference value.

As described above, the level of expression of IFIT1, IFIT2 and IFIT3 genes is measured in a biological sample from a subject suffering from a disease caused by a coronavirus infection, preferably from a mild to moderate form of COVID-19.

As used herein, "biological sample" refers to a biological sample isolated from a subject and can include, by way of example and not limitation, bodily fluids, cell samples and/or tissue extracts such as homogenates or solubilized tissue obtained from a subject.

In one embodiment, the present invention does not comprise obtaining a biological sample from a subject. Thus, in one embodiment, the biological sample from the subject is a biological sample previously obtained from the subject. Said biological sample may be conserved in adequate conditions before being used as described herein.

In one embodiment, the biological sample from the subject is a cell sample and/or tissue extract, preferably a nasal epithelial cells sample. Methods for obtaining a cell sample and/or tissue extract, especially a nasal epithelial cells sample, are routinely used in clinical laboratories.

According to the present invention, the level of expression of IFIT1, IFIT2 and IFIT3 genes may be measured by any known method in the art. Methods for measuring an expression level are well-known to the skilled artisan. For example, the level of expression of IFIT1, IFIT2 and IFIT3 genes can be evaluated by RT-PCR or SYBR green PCR analyses. Especially, the level of expression of IFIT1, IFIT2 and IFIT3 genes can be evaluated using the method used in Example 3.

According to one embodiment, the reference value is a reference level of expression of one or more of IFIT1, IFIT2 and IFIT3 genes derived from a reference population. In one embodiment, the reference value is derived from population studies, including, for example, subjects having a similar age range, or subjects in the same or similar ethnic group.

According to one embodiment, the reference value is derived from the measure of the level of expression of IFIT1, IFIT2 and/or IFIT3 genes in a biological sample obtained from one or more subjects who are substantially healthy. In one embodiment, a "substantially healthy subject" is a subject who has not been diagnosed or identified as having or suffering from a disease caused by a coronavirus. In one embodiment, a "substantially healthy subject" is a subject who has not been diagnosed or identified as having or suffering from a disease caused by a coronavirus or any other infection. Thus, in one embodiment, the reference value is a reference level of expression of IFIT1, IFIT2 and/or IFIT3 genes, derived from a reference population of subjects who are substantially healthy.

According to another embodiment, the reference value is derived from the measure of the level of expression of IFIT1, IFIT2 and/or IFIT3 genes in a biological sample from one or more subjects diagnosed or identified as suffering, or having suffered, from a disease caused by a coronavirus, in particular from COVID-19 caused by SARS-CoV-2. Thus, in one embodiment, the reference value is a reference level of expression of IFIT1, IFIT2 and/or IFIT3 genes derived from a reference population of subjects diagnosed or identified as suffering, or having suffered, from a disease caused by a coronavirus, in particular from COVID-19 caused by SARS-CoV-2. In such case, the reference value can be derived from statistical analyses and/or risk prediction data of a reference population as described hereinabove obtained from mathematical algorithms and computed indices of a disease caused by a coronavirus, in particular COVID-19 caused by SARS-CoV-2.

In one embodiment, a level of expression of IFIT1, IFIT2 and/or IFIT3 genes, measured in the biological sample from the subject, lower than the reference value as described hereinabove indicates that the subject as described hereinabove is susceptible to respond to a therapy by a prostaglandin receptor agonist, preferably an EP2 and/or EP4 agonist. By "lower" is it meant a level of expression of IFIT1, IFIT2 and/or IFIT3 genes which is at least 2 fold less than the reference value, i.e. a downregulation by at least 2 fold.

### Therapeutic effect

In one embodiment, the use of a prostaglandin receptor agonist as described above is of particular interest within the first 10 days after the infection by a coronavirus, especially SARS-CoV-2; preferably within the first 8 days after the infection; more preferably within the first 6 days after the infection.

In one embodiment, the use of a prostaglandin receptor agonist as described above prevents the onset of a severe form and/or a complication of COVID-19.

In one embodiment, the use of a prostaglandin receptor agonist as described above prevents progressive status degradation of the subject.

In one embodiment, the use of a prostaglandin receptor agonist as described above prevents the acute status degradation of the subject.

In one embodiment, the use of a prostaglandin receptor agonist as described above prevents further global and respiratory status degradation. In the meaning of the invention, global and respiratory status degradations include, but are not limited to, thrombotic complications, pulmonary embolism, cardiovascular failure, renal failure, liver failure, secondary infection or sepsis.

In one embodiment, the use of a prostaglandin receptor agonist as described above preserve and/or restore mucociliary cilia dynamic, especially in epithelial cells. This present advantage to limit the entry of the virus and reduce anosmia.

In one embodiment, the use of a prostaglandin receptor agonist as described above reduces ACE2 expression. This present the advantage to enable to reduce viral entry.

In one embodiment, the use of a prostaglandin receptor agonist as described above increase IFNg production. This is of particular interest at early stage of the infection in order to restore mediating molecules of innate immunity, thereby avoiding a subsequent massive cytokine storm counter reaction.

In one embodiment, the use of a prostaglandin receptor agonist as described above prevents hyperinflammation and/or cytokine storm.

In one embodiment, the use of a prostaglandin receptor agonist as described above prevents the clinical progression of the disease.

In one embodiment, the subject is considered treated if said subject does not progress to severe respiratory distress following the administration of the prostaglandin receptor agonist. In one embodiment, the subject is considered treated if said subject does not progress to severe respiratory distress after 1, 2, 3, 4, 5, 6, 7, 8, 9, 10, 11, 12, 13, 14, 15, 16, 17, 18, 19 or 20 days (preferably consecutive days) of administration of the prostaglandin receptor agonist.

In one embodiment, progression to severe respiratory distress is assessed by a respiratory rate (RR) equal or superior to 30 breaths per minute, an oxygen saturation (SpO₂) equal or inferior to 93% in resting sate, and/or a ratio of arterial oxygen partial pressure (PaO₂) to fractional inspired oxygen (FiO₂) (PaO₂/FiO₂) equal or inferior to 300 mmHg.

In one embodiment, the subject is considered treated if said subject presents a decreased score on the WHO COVID ordinal scale. In one embodiment, the subject is considered treated if said subject presents a decreased score on the WHO COVID ordinal scale after 1, 2, 3, 4, 5, 6, 7, 8, 9, 10, 11, 12, 13, 14, 15, 16, 17, 18, 19 or 20 days (preferably consecutive days) of administration of the prostaglandin receptor agonist.

In one embodiment, the subject is considered treated if said subject does not progress to one of the following: admission to intensive care unit (ICU), invasive mechanical ventilation (with intubation) following the administration of the prostaglandin receptor agonist. In one embodiment, the subject is considered treated if said subject does not progress to one of the following: admission to ICU, invasive mechanical ventilation (with intubation), after 1, 2, 3, 4, 5, 6, 7, 8, 9, 10, 11, 12, 13, 14, 15, 16, 17, 18, 19 or 20 days (preferably consecutive days) of administration of the prostaglandin receptor agonist.

### Combination with other active agents

In one embodiment, the prostaglandin receptor agonist for use of the present invention is for administration with at least one further pharmaceutically active agent.

Examples of further pharmaceutically active agents that may be administered to a subject suffering from a disease caused by a coronavirus, such as COVID-19, as described hereinabove include, but are not limited to, anti-viral agents, anti-interleukin 6 (anti-IL-6) agents and other agents such as chloroquine or hydroxychloroquine.

In one embodiment, the at least one further pharmaceutically active agent is an antiviral agent, an anti-IL-6 agent, chloroquine, hydroxychloroquine, or any mixes thereof.

Example of antiviral agents that may be administered to a subject suffering from a disease caused by a coronavirus as described hereinabove include, without being limited to, remdesivir, and a combination of lopinavir and ritonavir (lopinavir/ritonavir).

In one embodiment, the at least one further pharmaceutically active agent is remdesivir, or a combination of lopinavir and ritonavir (lopinavir/ritonavir).

As used herein, anti-IL-6 agents target either IL-6 (interleukin 6 or interleukin-6) or its receptor (IL-6R). Example of anti-IL-6 agents that may be administered to a subject suffering from a disease caused by a coronavirus as described hereinabove include, without being limited to, tocilizumab and sarilumab.

In one embodiment, the at least one further pharmaceutically active agent is tocilizumab or sarilumab.

In one embodiment, the at least one further pharmaceutically active agent is selected from the group comprising or consisting of remdesivir, a combination of lopinavir and ritonavir, tocilizumab, sarilumab, chloroquine, hydroxychloroquine, and any mixes thereof.

According to the present invention, the prostaglandin receptor agonist as described hereinabove, may be said at least one further pharmaceutically active agent.

In one embodiment, the subject receives a prostaglandin receptor agonist as described hereinabove as part of a treatment protocol. In one embodiment, said treatment protocol further comprises, before, concomitantly or after the administration of the prostaglandin receptor agonist, the administration of another pharmaceutically active agent, as described herein. Therefore, the subject to be treated was previously treated or is to be treated with another pharmaceutically active agent.

Another object of the invention is thus a kit-of-part comprising, in a first part, at least one a prostaglandin receptor agonist as described hereinabove and, in a second part, another pharmaceutically active agent, as described hereinabove.

### Administration & doses

According to one embodiment, the prostaglandin receptor agonists may be administered by oral, parenteral (e.g., intramuscular, intraperitoneal, intravenous, ICV, intracisternal injection or infusion, subcutaneous injection, or implant), by inhalation spray, nasal, rectal, sublingual, or topical routes of administration and may be formulated in suitable dosage unit formulations containing conventional non-toxic pharmaceutically acceptable carriers, adjuvants and vehicles appropriate for each route of administration.

The pharmaceutical compositions for the administration of the compounds of this invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

In one embodiment, the prostaglandin receptor agonist for use in the present invention is administered by oral route. In one embodiment, the prostaglandin receptor agonist is formulated as a pharmaceutical composition containing the prostaglandin receptor agonist in a form suitable for oral use, for example, as tablets, troches, lozenges, aqueous or oily suspensions, dispersible powders or granules, emulsions, hard or soft capsules, or syrups or elixirs.

In one embodiment, the prostaglandin receptor agonist for use in the present invention is administered by injection. In one embodiment, the prostaglandin receptor agonist is formulated as a pharmaceutical composition containing the prostaglandin receptor agonist in a form suitable for injection. In one embodiment, the prostaglandin receptor agonist administered by infusion, preferably by intravenous infusion. In another embodiment, the prostaglandin receptor agonist is injected intraperitoneally. In another embodiment, the prostaglandin receptor agonist invention is injected intradermally.

The pharmaceutical compositions may be in the form of a sterile injectable aqueous or oleaginous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids, such as oleic acid find use in the preparation of injectables.

In one embodiment, the prostaglandin receptor agonist for use in the present invention is administered by nasal route. The nasal route of administration is of particular interest since ACE2 receptor is highly expressed in nasal epithelial cells (Hou et al., Cell, 2020). ACE2 appears to be used by SARS-CoV-2 as receptor for viral cell entry. Without willing to be bound by a theory, the prostaglandin receptor agonists such as EP2 and/or EP4 agonists, are effective by enabling to induce a reduction of the expression of ACE2 in epithelial and endothelial cells.

In one embodiment, the prostaglandin receptor agonist is formulated as a pharmaceutical composition containing the prostaglandin receptor agonist in a form suitable for nasal use, for example, to be delivered by a nebulizer/atomizer, a dry powder inhaler, a nasal inhaler or a metered-dose aerosol inhaler. Drugs delivered via a nebulizer/atomizer are generally formulated as sterile aqueous solutions (or suspensions).

In one embodiment, a dose of the prostaglandin receptor agonist as described herein ranging from about 0.005 µg to about 150 mg is administered (or is to be administered) to the patient.

In one embodiment, the prostaglandin receptor agonist is administered by intravenous infusion, preferably at a dose ranging from 0.005 µg/kg/min to 0.1 µg/kg/min.

In one embodiment, the prostaglandin receptor agonist is administered by intravenous injection at a dose ranging from 10 µg to 500 µg, preferably at a dose of 20 µg, 50 µg, 100 µg, 200 µg, 300 µg or 400 µg.

In one embodiment, the prostaglandin receptor agonist is administered by intravenous injection at a dose ranging from 0.1 µg to 5 µg, preferably 5 µg, to reach stable 0.1 µg/kg/min.

In one embodiment, the prostaglandin receptor agonist is administered by intravenous injection at a dose ranging from 1 µg to 40 µg, preferably 40 µg, twice daily over 2 hours preferably in 50 to 150 ml isotonic sodium chloride solution.

In one embodiment, the prostaglandin receptor agonist is administered by inhalation at a dose ranging from 1 ng/kg/min to 300 ng/kg/min, preferably from 150 ng/kg/min to 300 ng/kg/min.

In one embodiment, the prostaglandin receptor agonist is orally administered at a dose ranging from 4 µg to 400 µg, preferably from 25 µg to 400 µg.

In one embodiment, the prostaglandin receptor agonist is administered by a nasal spray at a dose of about 100 mg per dose.

In one embodiment, the prostaglandin receptor agonist is to be administered as a single dose. In another embodiment, the prostaglandin receptor agonist is to be administered as repeated doses, such as, for example, 4 times a day, 3 times a day, 2 times a day, once every 24 hours (i.e., once a day), once every two days, 3 times a week, 2 times a week or once a week, preferably once every 24 hours or once every two days.

In one embodiment, the prostaglandin receptor agonist is to be administered during 1, 2, 3, 4, 5, 6, 7, 8, 9, 10, 11, 12, 13, 14, 15, 16, 17, 18, 19 or 20 days (preferably consecutive days).

In one embodiment, the prostaglandin receptor agonist for use in the present invention is to be administered once every 24 hours during 2, 3, 4, 5, 6, 7, 8, 9, 10, 11, 12, 13, 14, 15, 16, 17,18, 19 or 20 consecutive days, preferably during at least 3 consecutive days.

### Method of treatment / Use for the manufacture of a medicament

This invention thus relates to the use of prostaglandin receptor agonists in the treatment of a disease caused by a coronavirus infection as described hereinabove.

This invention also relates to the use of prostaglandin receptor agonists in the manufacture of a medicament for the treatment of a disease caused by a coronavirus infection as described hereinabove.

This invention also relates to a method for the treatment of a disease caused by a coronavirus infections as described hereinabove, preferably COVID-19 caused by SARS-CoV-2, in a subject in need thereof, comprising a step of administrating to said subject a therapeutically effective amount of a prostaglandin receptor agonist.

In one embodiment, the method of the invention comprises administering at least one further pharmaceutically active agent as described hereinabove.

Another object of the present invention is a pharmaceutical composition for treating or for use in the treatment of a disease caused by a coronavirus, preferably COVID-19 caused by SARS-CoV-2, in a subject in need thereof, said pharmaceutical composition comprising a prostaglandin receptor agonist as described hereinabove and at least one pharmaceutically acceptable excipient.

Another object of the present invention is the use of a prostaglandin receptor agonist as described hereinabove for the manufacture of a medicament for the treatment of a disease caused by a coronavirus, preferably COVID-19 caused by SARS-CoV-2, in a subject in need thereof.

### EXAMPLES

The present invention is further illustrated by the following examples.

### Example 1: Prostaglandin receptor agonists restore mucociliary cilia dynamic

***Purpose.*** This example aims at demonstrating that prostaglandin receptor agonists, especially EP2 agonists, can prevent the degradation of nasal epithelial cilia induced by SARS-Cov-2 and restore the ciliogenesis, on a cellular model modeling ciliogenesis deficiency.

### Materials and Methods

*Cell culture, compound treatment and immunofluorescence.* Patient and control cells (typically at 10^5 cells/well) were seeded in 96 well glass microplates (typically Sensoplates, Greiner) at 39°C. On the third day of culture, cells were incubated for 48 h. Drugs previously identified and selected were tested. For each drug, typically three to five doses were tested, range chosen depending on the live-cell cytotoxicity assay. Most if not all drugs were dissolved in DMSO and dilutions were made to have the same final percentage of DMSO (from 0.04% to 0.1%, depending on the drug).

Cells were fixed after 5 days of culture in cold methanol for 5 min, then typically treated with PBS-0.1% Tween 20-1% BSA for approximately 40 min, before incubating with primary antibodies (typically for ciliary marker, with rabbit anti-ARL13b (17711-1-AP, Euromedex), mouse anti-gamma tubulin (T6557, Sigma) and DAPI (62247, ThermoFisher Scientific)) for 1 h at room temperature, and then with appropriate fluorescent secondary antibody.

*Data acquisition, quantification and analysis.* Images were acquired within three days using the Opera Phenix (typically with an objective 40x air, PerkinElmer). Automated acquisition with Z-stack (typically ranging 5 to 40 per field) per well was performed. Simultaneous four camera and five lasers to be able to acquire different wavelength at the same time to speed up acquisition.

The number of ciliated cells was measured using a semi-automated analysis on the Harmony^{®} software (PerkinElmer). Briefly, images were analyzed using the building blocks approach in Harmony^{®} software to detect and segment the nucleus (following DAPI staining) and find within a 20 px enlarged region the basal body. Then the area around the basal body was enlarged by 20 px to detect green signal corresponding to the primary cilia. With conventional filters (intensity, size...) the software segmented candidate primary cilium. Hundreds of phenotypic parameters were calculated for every candidate cilia using SER texture (intensity patterns) and advanced STAR morphology parameters (distribution of either texture features or fluorescence intensities inside a region of interest). Using the PhenoLOGIC^{™} (PerkinElmer) machine-learning option in Harmony^{®} (PerkinElmer), the parameters best suited to discriminate cilia were defined by supervised machine learning and used to obtain final detection and counting of primary cilium. Because of high cell confluence, harmony software was not able to perform good nucleus segmentation. The number of basal bodies was considered for cell numbers as there is only one basal body per cell in our cell culture condition.

### Example 2: Prostaglandin receptor agonists alter the expression of the specific cellular entry receptor ACE2 of SARS-CoV-2

***Purpose.*** This example aims at demonstrating that prostaglandin receptor agonists, especially EP2 agonists, downregulate the expression of ACE2 on a model of human epithelial NSLC cell lines A549 and human primary ciliated cell prepared with a gentle nasal brushing. By downregulating ACE2 expression, prostaglandin receptor agonists, especially EP2 agonists, enable to considerably reduce viral entry, and especially limit expansion at the vascular level. In the specific case of SARS-Cov-2, ACE2 is not only a receptor to the virus, but may also contribute to post infection regulation, immune response, cytokine secretion, and viral genome replication. Reduction of ACE2 thus has also additional benefit during the recovery phase of COVID-19.

### Materials and Methods

Human epithelial NSLC cell lines A549 were purchased from ATCC (catalogue number: ATCC CCL-185, American Type Culture Collection (ATCC), Manassas, VA). A549 cells were maintained in DMEM containing 10% fetal bovine serum (FBS), 4 mM GlutaMax (Thermo Scientific, Waltham, MA), 500 µg/mL Normocin (InvivoGen, San Diego, CA), 100 units/mL penicillin, and 100 mg/mL streptomycin (Thermo Scientific).

Human primary ciliated cells obtained by gentle nasal brushing cells (GNBC cells) are collected from different patient sources, as described by Veit et al. (Nat. Med., 2018, 24(11), 1732-1742). Ciliogenesis is performed on cells plated on to poly-lysine-coated coverslips in 3.5-cm plates at 0.4 × 10^6 cells per well, allowed to attach for 24 hr, and then serum starved for 48 hr.

Gene downregulation of ACE2 has been evaluated with by RT-PCR and SYBR green PCR analyses. Total RNA was extracted from the cells or tissues and purified with the TRIzol reagent according to the manufacturer's protocol (Invitrogen). RNA was reverse transcribed by SuperScript^{®} III reverse transcriptase (Invitrogen). The genes were amplified by with specific primers designed using BLAST alignment. Three housekeeping gene, GAPDH, β-actin gene, and rare PBGD (porphobilinogen deaminase) gene were combined for normalization. Relative expression levels have been evaluated with the parameter of (ΔCt) of different human cell lines and human GNB cells after treatment with EP2, EP4 or EP2/EP4 agonists at a dose response concentration varying from 10E-2 to 10E2 fold of their respective EC50 of binding activity on each relevant EP receptor.

Immunoblot analysis on A549 cells treated with different EP2 and/or EP4 agonists doses ranging from 10E-2 to 10E2 fold of their respective binding EC50 is performed by lysing cells as described by Jia et al. (J. Virol., 2005, 79(23), 14614-14621).

***Results.*** Alprostadil or/and Taprenepag significantly reduce the expression of mRNA and protein level of ACE2 in A549 and GNC cells after an incubation of 3h to 24h.

### Example 3: Prostaglandin receptor agonists restore the expression of ISG genes

***Purpose.*** This example aims at demonstrating that prostaglandin receptor agonists, especially EP2 agonists, significantly and specifically regulate the expression of genes related to interferon in human cells, especially interferon-stimulated genes (ISG). By increasing the expression of INF post viral infection, prostaglandin receptor agonists, especially EP2 agonists, lead to an observation of significant IFN release on immune cells involved in the innate response to infection by coronaviruses. Restoring the mediating molecules of innate immunity in the early stages of the infection more quickly trigger the adaptive immune response, necessary to control the progression of the viremia causing the explosive and deleterious cytokine response.

### Materials and Methods

*Cell Culture.* Immortalized temperature-sensitive URECs are maintained and resuspended in complete RCGM (RCGM medium (0.5% FBS), 1.5% (v/v) certified FBS). Cells were seeded at 116,000 cells/well (350k cells/cm²) in 200 µl total volume of complete RCGM medium. Seed in triplicates per each experimental condition. Incubate at 39°C to stop proliferation for 72 hours. After 72hrs of cell growth, remove complete RCGM media. Replace with fresh complete RCGM media (2% FBS) containing the drug (at either 0.2, 2, or 10 µM) or vehicle control (media alone or 0.04% DMSO) at the appropriate concentration.

*RNA Extraction.* Extract RNA using RNeasy Micro Kit (Qiagene) using supplier's procedure. In short, disrupt the cells by adding QIAzol Lysis Reagent. Scratch each well with the pipette tip and pipette to mix. Homogenize the cells by vortexing. Adjust sample volume. Homogenize by passing the lysate through a 20-gauge needle. Precipitate proteins using chloroform solution. Load into a RNeasy MinElute column. Wash and elute following supplier's procedure. Prepare sample at a concentration of 5ng/µl for quality control and measure by spectrometry (Xpose, Trinean) and confirming RNA as a RIN using capillary electrophoresis (TapeStation, Agilent). The average yield of extraction is 0.4 ± 0.2µg /100,000 cells (1 well of a 96-well plate).

*Microarray data analysis.* Affymetrix Human ClariomD were used following manufacturers protocols. Affymetrix Human ClariomD data were normalized using quantile normalization with adjustment based on the median intensity of probes with similar GC content (using Affymetrix Power Tools). Background correction was made using the antigenomic probes. Only probes targeting exons annotated from FAST DB v2016_1 transcripts were selected. Probes were considered as expressed if the DABG p-value ≤ 0.05 (Detection Above Background p-values were calculated using Affymetrix Power Tools) in more or equal than 60% of samples. Genes were considered as expressed if more or equal than 50% of their probes are expressed. Minimum 4 selected probes were required to assess gene expression. If possible only high-specific probes were selected (i.e., not overlapping with repeat regions; not cross-hybridizing; and 40 ≤ GC% ≤ 60). In addition, if possible, only probes targeting constitutive gene regions were selected (i.e., targeting at least 75% of transcripts of a given gene). We performed a paired Student's t-test to compare gene intensities in the different biological replicates. Genes were considered significantly regulated when fold-change was ≥ 1.5 and uncorrected p- value ≤ 0.05.

Analysis at the splicing level was first performed taking into account only exon probes ("EXON" analysis) in order to potentially detect new alternative events that could be differentially regulated (i.e., without taking into account known alternative events).

Analysis at the splicing level was also performed by taking into account known patterns ("PATTERN" analysis) using the FAST DB splicing patterns annotation (i.e., for each gene, all possible splicing patterns were defined by comparing exon content of transcripts). All types of alternative events can be analyzed: Alternative first exons, alternative terminal exons, cassette exon, mutually exclusive exons, alternative 5' donor splice site, alternative 3' -acceptor splice sites, intron retention, internal exon deletion and complex events corresponding to mix of several alternative event categories). "EXON" and "PATTERN" analyses were based on the splicing-index calculation. Results were considered statistically significant for uncorrected p-values ≤ 0.05 and fold-changes ≥ 2.0. Finally, significant results from "EXON" and "PATTERN" analyses were merged to obtain a single result list.

*Unsupervised analysis.* The PCA has been performed using "prcomp" function in R and the 2 first dimensions were plotted. The clustering has been performed using using "dist" and "hclust" functions in R, using Euclidean distance and Ward agglomeration method. Bootstraps have been realized using "pvclust" package in R, with the same distance and agglomeration method, using 1000 bootstraps.

*Sample reproducibility study.* Pearson correlation tests have been performed for each pair of samples using "cor.test" function in R. Heatmaps and clusterings were performed with "dist" and "hclust" functions in R using Euclidean distance and Ward agglomeration method. Bootstraps were realized as described in "Unsupervised analysis" method.

*Pathway*/*Gene Ontology (GO) analysis.* Analysis for enriched REACTOME pathways and GO terms were performed using DAVID Functional annotation Tool (v6.8). GO terms and pathways were considered as enriched if fold enrichment ≥ 2.0, uncorrected p-value ≤ 0.05 and minimum number of regulated genes in pathway/term ≥ 2.0. Analysis was performed three times: using all regulated genes, using up-regulated genes and using down- regulated genes only. Union of these three analyses was made to provide a single list of results.

### Results

Pharmacological treatment of UREC human cells with PGE1 significantly modifies the expression of a major biological pathway related to viral infection and interferon signaling. The relevance of the modification of this pathway has been assessed with the unsupervised analysis, and the gene ontology approach. Interestingly, induction of IFN related gene appears to be consistent regardless the different clinically relevant doses tested (from 0.2 to 10 microM).

For instance, 3 different IFN related genes overexpressed with a fold changes >3: IFIT1, interferon induced protein with tetratricopeptide repeats 1, IFIT2, interferon induced protein with tetratricopeptide repeats 2 and IFIT3 interferon induced protein with tetratricopeptide repeats 3. Other genes have been identified.

The significance of the pathways modified with the modification the interferon genes is listed in the table 2.The complete list is represented in the table 3. The significantly enriched GO terms are listed in table 4.

**Table 2: Subselection list of the most statistically relevant Reactome pathway terms identified by the comparison of UREC cells transcriptome after treatment by 2 micromolar of PGE1 versus non treated control.**

| REACTOME Pathway | Pathway Description (REACTOME) | Nb Genes in Pathway | Nb Regulated Genes (Up/Down) | Min P-Value (Adjusted) |
|---|---|---|---|---|
| R-HSA-909733 | Interferon alpha/beta signaling | 67 | 20 (20/0) | 4.45E-13 |
| R-HSA-1169408 | ISG15 antiviral mechanism | 73 | 11 (11/0) | 2.10E-03 |
| R-HSA-168928 | RIG-I/MDA5 mediated induction of IFN-alpha/beta pathways | 17 | 6 (6/0) | 5.55E-03 |
| R-HSA-877300 | Interferon gamma signaling | 88 | 11 (11/0) | 5.54E-03 |
| R-HSA-918233 | TRAF3-dependent IRF activation pathway | 14 | 4 (4/0) | 2.21E-01 |
| R-HSA-936440 | Negative regulators of RIG-I/MDA5 signaling | 34 | 5 (5/0) | 3.71E-01 |
| R-HSA-5676594 | TNF receptor superfamily (TNFSF) members mediating non-canonical NF-kB pathway | 18 | 4 (3/1) | 5.93E-01 |
| R-HSA-933541 | TRAF6 mediated IRF7 activation | 33 | 4 (4/0) | 7.96E-01 |

**Table 3: List of the most statistically relevant genes overexpressed in UREC cells transcriptome after treatment by 2 micromolar of PGE1 versus non treated control.**

| Pathway Description (REACTOM E) | FAST DB STABLE ID | Gene Symbol | Gene Name | Regul ation | Fold-Change | P-Valu e |
|---|---|---|---|---|---|---|
| Interferon alpha/beta signaling | GSHG000 3550 | IFIT1 | interferon induced protein with tetratricopeptide repeats 1 | up | 5.18 | 1.22 |
| | | | | | | E-02 |
| ISG15 antiviral mechanism | GSHG000 3550 | IFIT1 | interferon induced protein with tetratricopeptide repeats 1 | up | 5.18 | 1.22 |
| | | | | | | E-02 |
| Interferon alpha/beta signaling | GSHG000 3547 | IFIT2 | interferon induced protein with tetratricopeptide repeats 2 | up | 4.84 | 9.20 |
| | | | | | | E-03 |
| Interferon alpha/beta signaling | GSHG001 9499 | MX2 | MX dynamin like GTPase 2 | up | 4.02 | 1.85 |
| | | | | | | E-02 |
| ISG15 antiviral mechanism | GSHG001 9499 | MX2 | MX dynamin like GTPase 2 | up | 4.02 | 1.85 |
| | | | | | | E-02 |
| Interferon alpha/beta signaling | GSHG000 3548 | IFIT3 | interferon induced protein with tetratricopeptide repeats 3 | up | 3.99 | 1.37 |
| | | | | | | E-02 |
| Interferon alpha/beta signaling | GSHG001 6231 | RSAD2 | radical S-adenosyl methionine domain containing 2 | up | 3.79 | 9.14 |
| | | | | | | E-03 |
| Interferon alpha/beta signaling | GSHG001 9500 | MX1 | MX dynamin like GTPase 1 | up | 3.68 | 7.10 |
| | | | | | | E-03 |
| ISG15 antiviral mechanism | GSHG001 9500 | MX1 | MX dynamin like GTPase 1 | up | 3.68 | 7.10 |
| | | | | | | E-03 |
| Interferon alpha/beta signaling | GSHG000 7017 | --- | --- | up | 3.58 | 6.92 |
| | | | | | | E-03 |
| Interferon gamma signaling | GSHG000 7017 | --- | --- | up | 3.58 | 6.92 |
| | | | | | | E-03 |
| ISG15 antiviral mechanism | GSHG003 0744 | DDX58 | DEXD/H-box helicase 58 | up | 2.76 | 2.18 |
| | | | | | | E-02 |
| RIG-I/MDA5 mediated induction of IFN-alpha/beta pathways | GSHG003 0744 | DDX58 | DEXD/H-box helicase 58 | up | 2.76 | 2.18 |
| | | | | | | E-02 |
| TRAF3 - dependent IRF activation pathway | GSHG003 0744 | DDX58 | DEXD/H-box helicase 58 | up | 2.76 | 2.18 |
| | | | | | | E-02 |
| Negative regulators of RIG-I/MDA5 signaling | GSHG003 0744 | DDX58 | DEXD/H-box helicase 58 | up | 2.76 | 2.18 |
| | | | | | | E-02 |
| TRAF6 mediated IRF7 activation | GSHG003 0744 | DDX58 | DEXD/H-box helicase 58 | up | 2.76 | 2.18 |
| | | | | | | E-02 |
| Interferon alpha/beta signaling | GSHG011 8824 | IFI6 | interferon alpha inducible protein 6 | up | 2.51 | 1.88 |
| | | | | | | E-03 |
| RIG-I/MDA5 mediated induction of IFN-alpha/beta pathways | GSHG001 8106 | IFIH1 | interferon induced with helicase C domain 1 | up | 2.45 | 1.60 |
| | | | | | | E-02 |
| TRAF3 - dependent IRF activation pathway | GSHG001 8106 | IFIH1 | interferon induced with helicase C domain 1 | up | 2.45 | 1.60 |
| | | | | | | E-02 |
| Negative regulators of RIG-I/MDA5 signaling | GSHG001 8106 | IFIH1 | interferon induced with helicase C domain 1 | up | 2.45 | 1.60 |
| | | | | | | E-02 |
| TRAF6 mediated IRF7 activation | GSHG001 8106 | IFIH1 | interferon induced with helicase C domain 1 | up | 2.45 | 1.60 |
| | | | | | | E-02 |
| ISG15 antiviral mechanism | GSHG002 2725 | HERC5 | HECT and RLD domain containing E3 ubiquitin protein ligase 5 | up | 2.37 | 2.37 |
| | | | | | | E-02 |
| RIG-I/MDA5 mediated induction of IFN-alpha/beta pathways | GSHG002 2725 | HERC5 | HECT and RLD domain containing E3 ubiquitin protein ligase 5 | up | 2.37 | 2.37 |
| | | | | | | E-02 |
| Negative regulators of RIG-I/MDA5 signaling | GSHG002 2725 | HERC5 | HECT and RLD domain containing E3 ubiquitin protein ligase 5 | up | 2.37 | 2.37 |
| | | | | | | E-02 |
| Interferon alpha/beta signaling | GSHG000 4490 | IFITM1 | interferon induced transmembrane protein 1 | up | 2.35 | 8.00 |
| | | | | | | E-05 |
| Interferon alpha/beta signaling | GSHG000 7863 | OASL | 2'-5'-oligoadenylate synthetase like | up | 2.27 | 8.98 |
| | | | | | | E-03 |
| Interferon gamma signaling | GSHG000 7863 | OASL | 2'-5'-oligoadenylate synthetase like | up | 2.27 | 8.98 |
| | | | | | | E-03 |
| ISG15 antiviral mechanism | GSHG001 9814 | USP18 | ubiquitin specific peptidase 18 | up | 2.26 | 2.98 |
| | | | | | | E-02 |
| Interferon gamma signaling | GSHG003 1011 | TRIM1 4 | tripartite motif containing 14 | up | 2.02 | 2.40 |
| | | | | | | E-03 |
| Interferon alpha/beta signaling | GSHG000 7018 | --- | --- | up | 1.93 | 3.17 |
| | | | | | | E-02 |
| Interferon gamma signaling | GSHG000 7018 | --- | --- | up | 1.93 | 3.17 |
| | | | | | | E-02 |
| Interferon alpha/beta signaling | GSHG000 0017 | ISG15 | ISG15 ubiquitin-like modifier | up | 1.89 | 3.77 |
| | | | | | | E-02 |
| Interferon alpha/beta signaling | GSHG001 9164 | SAMH D1 | SAM and HD domain containing deoxynucleoside triphosphate triphosphohydrolas e 1 | up | 1.89 | 1.08 |
| | | | | | | E-02 |
| ISG15 antiviral mechanism | GSHG000 0017 | ISG15 | ISG15 ubiquitin-like modifier | up | 1.89 | 3.77 |
| | | | | | | E-02 |
| RIG-I/MDA5 mediated induction of IFN-alpha/beta pathways | GSHG000 0017 | ISG15 | ISG15 ubiquitin-like modifier | up | 1.89 | 3.77 |
| | | | | | | E-02 |
| Negative regulators of RIG-I/MDA5 signaling | GSHG000 0017 | ISG15 | ISG15 ubiquitin-like modifier | up | 1.89 | 3.77 |
| | | | | | | E-02 |
| Interferon alpha/beta signaling | GSHG000 5441 | IRF7 | interferon regulatory factor 7 | up | 1.88 | 4.76 |
| | | | | | | E-02 |
| Interferon gamma signaling | GSHG000 5441 | IRF7 | interferon regulatory factor 7 | up | 1.88 | 4.76 |
| | | | | | | E-02 |
| TRAF3-dependent IRF activation pathway | GSHG000 5441 | IRF7 | interferon regulatory factor 7 | up | 1.88 | 4.76 |
| | | | | | | E-02 |
| TRAF6 mediated IRF7 activation | GSHG000 5441 | IRF7 | interferon regulatory factor 7 | up | 1.88 | 4.76 |
| | | | | | | E-02 |
| Interferon gamma signaling | GSHG002 5634 | HLA-DRA | major histocompatibility complex, class II, DR alpha | up | 1.86 | 9.60 |
| | | | | | | E-03 |
| Interferon gamma signaling | GSHG000 2246 | GBP4 | guanylate binding protein 4 | up | 1.86 | 1.44 |
| | | | | | | E-02 |
| Interferon alpha/beta signaling | GSHG001 2173 | XAF1 | XIAP associated factor 1 | up | 1.82 | 1.69 |
| | | | | | | E-02 |
| ISG15 antiviral mechanism | GSHG001 7549 | EIF2A K2 | eukaryotic translation initiation factor 2 alpha kinase 2 | up | 1.74 | 8.60 |
| | | | | | | E-03 |
| Interferon alpha/beta signaling | GSHG001 8230 | STAT1 | signal transducer and activator of transcription 1 | up | 1.73 | 2.67 |
| | | | | | | E-02 |
| ISG15 antiviral mechanism | GSHG001 8230 | STAT1 | signal transducer and activator of transcription 1 | up | 1.73 | 2.67 |
| | | | | | | E-02 |
| Interferon gamma signaling | GSHG001 8230 | STAT1 | signal transducer and activator of transcription 1 | up | 1.73 | 2.67 |
| | | | | | | E-02 |
| ISG15 antiviral mechanism | GSHG002 1858 | EIF4E3 | eukaryotic translation initiation factor 4E family member 3 | up | 1.72 | 1.40 |
| | | | | | | E-04 |
| TNF receptor superfamily (TNFSF) members mediating non-canonical NF-kB pathway | GSHG000 5208 | BIRC3 | baculoviral IAP repeat containing 3 | down | 1.67 | 2.24 |
| | | | | | | E-03 |
| TNF receptor superfamily (TNFSF) members mediating non-canonical NF-kB pathway | GSHG000 8280 | TNFSF 13B | tumor necrosis factor superfamily member 13b | up | 1.65 | 1.56 |
| | | | | | | E-02 |
| Interferon alpha/beta signaling | GSHG001 2528 | IFI35 | interferon induced protein 35 | up | 1.62 | 4.72 |
| | | | | | | E-02 |
| Interferon alpha/beta signaling | GSHG000 2244 | GBP2 // GBP7 | guanylate binding protein 2 // guanylate binding protein 7 | up | 1.62 | 4.20 |
| | | | | | | E-03 |
| Interferon gamma signaling | GSHG000 2244 | GBP2 // GBP7 | guanylate binding protein 2 // guanylate binding protein 7 | up | 1.62 | 4.20 |
| | | | | | | E-03 |
| Interferon gamma signaling | GSHG000 2244 | GBP2 // GBP7 | guanylate binding protein 2 // guanylate binding protein 7 | up | 1.62 | 4.20 |
| | | | | | | E-03 |
| TNF receptor superfamily (TNFSF) members mediating non-canonical NF-kB pathway | GSHG001 2197 | TNFSF 12 // TNFSF 12-TNFSF 13 // TNFSF 13 | tumor necrosis factor superfamily member 12 // TNFSF12-TNFSF13 readthrough // tumor necrosis factor superfamily member 13 | up | 1.62 | 2.08 |
| | | | | | | E-03 |
| TNF receptor superfamily (TNFSF) members mediating non-canonical NF-kB pathway | GSHG001 2197 | TNFSF 12 // TNFSF 12-TNFSF 13 // TNFSF 13 | tumor necrosis factor superfamily member 12 // TNFSF12-TNFSF13 readthrough // tumor necrosis factor superfamily member 13 | up | 1.62 | 2.08 |
| | | | | | | E-03 |
| Interferon alpha/beta signaling | GSHG001 5611 | BST2 | bone marrow stromal cell antigen 2 | up | 1.6 | 4.25 |
| | | | | | | E-02 |
| Interferon alpha/beta signaling | GSHG000 9078 | IFI27 | interferon alpha inducible protein 27 | up | 1.57 | 4.07 |
| | | | | | | E-02 |
| ISG15 antiviral mechanism | GSHG001 3587 | MIR36 14 // TRIM2 5 | microRNA 3614 // tripartite motif containing 25 | up | 1.56 | 1.05 |
| | | | | | | E-02 |
| RIG-I/MDA5 mediated induction of IFN-alpha/beta pathways | GSHG001 3587 | MIR36 14 // TRIM2 5 | microRNA 3614 // tripartite motif containing 25 | up | 1.56 | 1.05 |
| | | | | | | E-02 |
| Interferon gamma signaling | GSHG001 3587 | MIR36 14 // TRIM2 5 | microRNA 3614 // tripartite motif containing 25 | up | 1.56 | 1.05 |
| | | | | | | E-02 |
| TRAF3 - dependent IRF activation pathway | GSHG001 3587 | MIR36 14 // TRIM2 5 | microRNA 3614 // tripartite motif containing 25 | up | 1.56 | 1.05 |
| | | | | | | E-02 |
| Negative regulators of RIG-I/MDA5 signaling | GSHG001 3587 | MIR36 14 // TRIM2 5 | microRNA 3614 // tripartite motif containing 25 | up | 1.56 | 1.05 |
| | | | | | | E-02 |
| TRAF6 mediated IRF7 activation | GSHG001 3587 | MIR36 14 // TRIM2 5 | microRNA 3614 // tripartite motif containing 25 | up | 1.56 | 1.05 |
| | | | | | | E-02 |
| RIG-I/MDA5 mediated induction of IFN-alpha/beta pathways | GSHG001 3412 | DHX58 | DEXH-box helicase 58 | up | 1.55 | 3.06 |
| | | | | | | E-02 |

**Table 4: GO Analysis on Regulated Genes. Subselection list of the most statistically relevant GO terms identified by the comparison of UREC cells transcriptome after treatment by 2 micromolar of PGE1 versus non treated control.**

| Term Type | GO ID Link | Go Term | Nb Genes in Term | Nb Regulated Genes (Up/Down) | Adjusted P-Value |
|---|---|---|---|---|---|
| biological process | GO:0060337 | type I interferon signaling pathway | 64 | 20 (20/0) | 2.19E-11 |
| biological process | GO:0051607 | defense response to virus | 165 | 27 (25/2) | 1.65E-09 |
| biological process | GO:0009615 | response to virus | 110 | 21 (19/2) | 3.09E-08 |
| biological _process | GO:0045071 | negative regulation of viral genome replication | 40 | 11 (11/0) | 8.43E-05 |
| biological process | GO:0045087 | innate immune response | 430 | 30 (27/3) | 6.61E-03 |
| biological process | GO:0006955 | immune response | 421 | 26 (19/7) | 6.66E-02 |
| biological process | GO:0071347 | cellular response to interleukin-1 | 71 | 9 (5/4) | 1.08E-01 |
| molecular function | GO:0005164 | tumor necrosis factor receptor binding | 29 | 6 (5/1) | 8.96E-02 |
| biological process | GO:0035455 | response to interferon-alpha | 10 | 4 (4/0) | 1.78E-01 |
| biological process | GO:0006954 | inflammatory response | 379 | 22 (12/10) | 1.93E-01 |
| biological _process | GO:0060333 | interferon-gamma-mediated signaling pathway | 71 | 8 (8/0) | 2.46E-01 |
| biological process | GO:0009597 | detection of virus | 5 | 3 (3/0) | 3.28E-01 |

## Claims

1. An EP2 and/or EP4 agonist for use in the treatment of a disease caused by a coronavirus infection in a subject in need thereof.

2. The EP2 and/or EP4 agonist for use according to claim 1, wherein the EP2 and/or EP4 agonist is selected from prostaglandin E1 (PGE1), prostaglandin E2 (PGE2), butaprost, butaprost free acid, ONO-AE1-259-01, taprenepag, taprenepag isopropyl, evatanepag, PGN-9856, omidenepag, 19-hydroxy-PGE2, 1-OH-PGE1, 11-deoxy-PGE2, 13, 14-dihydro-PGE1, L902688, CP734432, TCS 2510 and ONO-AE1-437.

3. The EP2 and/or EP4 agonist for use according to claim **1** or claim **2**, wherein the agonist is an EP2 agonist selected from prostaglandin E1 (PGE1), prostaglandin E2 (PGE2), butaprost, butaprost free acid, ONO-AE1-259-01, taprenepag, taprenepag isopropyl, evatanepag, PGN-9856, omidenepag, 19-hydroxy-PGE2.

4. The EP2 and/or EP4 agonist for use according to any one of claims **1** to **3**, wherein the agonist is prostaglandin E1 (PGE1), taprenepag or taprenepag isopropyl.

5. The EP2 and/or EP4 agonist for use according to any one of claims **1** to **4**, wherein the coronavirus is selected from HCoV-229E, HCoV-NL63, HCoV-OC43, HCoV-HKU1, MERS-CoV, SARS-CoV-1 and SARS-CoV-2; preferably the coronavirus is selected from MERS-CoV, SARS-CoV-1 and SARS-CoV-2; more preferably the coronavirus is SARS-CoV-2 and the disease caused by the coronavirus infection is coronavirus disease 2019 (COVID-19).

6. The EP2 and/or EP4 agonist for use according to any one of claims **1** to **5**, wherein the subject is infected by SARS-CoV-2 from less than 10 days, preferably from less than 8 days, more preferably from less than 6 days.

7. The EP2 and/or EP4 agonist for use according to any one of claims **1** to **6**, wherein the subject suffers from a mild or moderate form of COVID-19.

8. The EP2 and/or EP4 agonist for use according to any one of claims **1** to **7**, wherein the subject is at risk to develop a severe form and/or a complication of COVID-19.

9. The EP2 and/or EP4 agonist for use according to claim **7** or claim **8**, wherein the severe form and/or the complication of COVID-19 is selected from respiratory failure, including acute respiratory failure or acute respiratory distress syndrome (ARDS); persistence of respiratory failure including the requirement for prolonged mechanical ventilation, in particular prolonged mechanical ventilation lasting more than 15 days, and failed extubation; secondary infection or superinfection; thrombotic complications including venous and/or arterial thromboembolism; pulmonary embolism; cardiocirculatory failure (which may also be referred to as cardiovascular failure); renal failure including acute kidney injury (AKI); liver failure; and any combinations thereof.

10. The EP2 and/or EP4 agonist for use according to any one of claims **1** to **9**, wherein the subject present one or more of the following risk factors:
- the subject is older than 60, 65, 70, 75, 80 or 85 years of age;
- the subject suffers from at least one comorbidity selected from acute kidney injury, asthma, atopy, autoimmune or auto-inflammatory diseases or conditions, bone marrow or stem cell transplantations in the past 6 months, bronchial hyperreactivity, cardiovascular diseases or conditions, chronic bronchitis, chronic kidney diseases, chronic liver disease, chronic obstructive pulmonary disease (COPD), ciliary deficiencies (hereditary or acute ciliary deficiencies), cystic fibrosis, diabetes, emphysema, hematological diseases, high blood pressure, immunodeficiency, infection with HIV, malignancy (*i.e*., cancer), obesity, pulmonary hypertension, rare diseases and inborn errors of metabolism that significantly increase the risk of infections (such as severe combined immunodeficiency), reactive airway disease, recipient of solid organ transplants, and severe respiratory conditions;
- the subject receives or has recently received one or more of the treatments selected from active chemotherapy or radical radiotherapy for lung cancer, immunosuppression therapy in particular immunosuppression therapy sufficient to significantly increase the risk of infection, immunotherapy or antibody treatment for cancer, and targeted cancer treatments that can affect the immune system (such as protein kinase inhibitors or PARP inhibitors);
- the subject has one or more of the habits or behaviors selected from active smoking, chronic passive smoking (also referred to as environmental exposure smoking).

11. The EP2 and/or EP4 agonist for use according to any one of claims **1** to **10**, wherein the subject presents low early IFN-gamma response.

12. The EP2 and/or EP4 agonist for use according to any one of claims **1** to **11**, wherein the EP2 and/or EP4 agonist is to be administered simultaneously, separately or sequentially with at least one further pharmaceutically active agent selected from anti-viral agents, anti-interleukin 6 (anti-IL-6) agents, other agents such as chloroquine or hydroxychloroquine, and any mixtures thereof.

13. A method for identifying a subject suffering from a disease caused by a coronavirus infection, preferably from a mild to moderate form of COVID-19, susceptible to respond to an EP2 and/or EP4 agonist, said method comprising:
- measuring the level of expression of ACE2 in a biological sample from the subject; and
- comparing the level of expression of ACE2 measured in the biological sample from the subject to a reference value.

14. A method for identifying a subject suffering from a disease caused by a coronavirus infection, preferably from a mild to moderate form of COVID-19, susceptible to respond to an EP2 and/or EP4 agonist, said method comprising:
- measuring the level of expression of one or more of IFIT1, IFIT2 and IFIT3 genes in a biological sample from the subject; and
- comparing the level of expression of one or more of IFIT1, IFIT2 and IFIT3genes measured in the biological sample from the subject to a reference value.

15. An EP2 and/or EP4 agonist for use in a method to regulate interferon signaling pathway in a subject in need thereof; preferably to induce one or more of IFIT1, IFIT2 and IFIT3 genes in a subject in need thereof.
